# EUROPÄISCHE PATENTANMELDUNG

(11) **EP 0 628 556 A1**
(43) Veröffentlichungstag der Anmeldung: **14.12.1994**
(21) Anmeldenummer: 94108296.8
(22) Anmeldetag: 30.05.1994
(51) Int. Cl.: C07D 471/04, A61K 31/435

(54) **Imidazopyridine**

(30) Priorität: 07.06.1993 DE 4318813
(71) Anmelder: MERCK PATENT GmbH, D-64293 Darmstadt (DE)
(72) Erfinder: Oswald, Mathias, Dr., D-64673 Zwingenberg (DE); Mederski, Werner, Dr., D-64390 Erzhausen (DE); Dorsch, Dieter, Dr., D-64372 Ober-Ramstadt (DE); Schelling, Pierre, Prof. Dr., D-64354 Mühltal (DE); Beier, Norbert, Dr., D-64354 Reinheim (DE); Lues, Ingeborg, Dr., D-64297 Darmstadt (DE); Minck, Klaus-Otto, dr., D-64372 Ober-Ramstadt (DE)

(57) **Zusammenfassung**

Neue Imidazopyridinderivate der Formel I 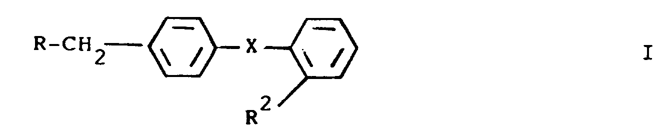
worin
- R: 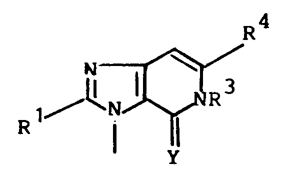
bedeutet und R¹, R², R³, R⁴, X und Y die in Patentanspruch 1 angegebenen Bedeutungen haben, sowie deren Salze zeigen Angiotensin II-antagonistische Eigenschaften und können zur Behandlung von Hypertension, Aldosteronismus, Herzinsuffizienz und erhöhtem Augeninnendruck sowie von Störungen des Zentralnervensystems verwendet werden.

## Beschreibung

Die Erfindung betrifft neue Imidazopyridinderivate der Formel I 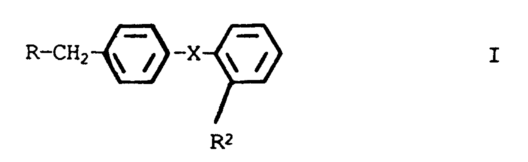
worin
- R: 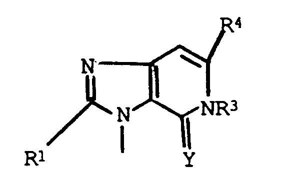
- R¹: A, C₂-C₆-Alkenyl, C₂₋C₆-Alkinyl, C₃-C₈-Cycloalkyl-CₖH₂ₖ- oder C₁-C₆-Alkyl, worin eine CH₂-Gruppe durch O oder S ersetzt ist,
- R²: -SO₂NH-COOR⁵, -SO₂NH-COR⁵, -SO₂NH-SO₂R⁵, -SO₂NH-CONR⁵R⁶, -C(NH₂)=NOH,
4,5-Dihydro-5-oxo-1,2,4-oxadiazol-3-yl,
4,5-Dihydro-5-thioxo-1,2,4-oxadiazol-3-yl,
2-Oxo-3H-1,2,3,5-oxathiadiazol-4-yl,
2,2-Dioxo-3H-1,2,3,5-oxathiadiazol-4-yl,
2,3-Dihydro-3-oxo-1,2,4-oxadiazol-5-yl,
2,5-Dihydro-2,5-dioxo-1H-imidazol-4-yl,
4,5-Dihydro-5-oxo-1H-1,2,4-triazol-3-yl,
4,5-Dihydro-5-thioxo-1H-1,2,4-triazol-3-yl,
2,3-Dihydro-2-oxo-1,3,4-thiadiazol-5-yl oder
4,5-Dihydro-5-oxo-1,2,4-thiadiazol-3-yl,
- R³: H, R¹¹, unsubstituiertes oder ein- oder mehrfach durch COOH, COOA, CN, NO₂, NR⁹R¹⁰, NHCOR¹¹, NHSO₂R¹¹, Hal und/oder Ar substituiertes C₂-C₆-Alkenyl, C₂-C₆-Alkinyl, -CₙH₂ₙ-R¹², -CHR¹³-CₖH₂ₖ-R¹⁴ oder 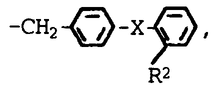
- R⁴: H oder Hal,
- R⁵ und R⁶: jeweils H, eine C₁-C₆-Alkylgruppe, worin auch eine CH₂-Gruppe durch O oder S ersetzt sein kann oder eine zusätzliche C-C-Doppelbindung enthalten sein kann und welche zusätzlich durch OH, OR⁷, Ar, Het², NR⁷R⁸, NR⁷-COOR⁸, NR⁷-COO-CₜH₂ₜ-Ar, NR⁷-COO-CₜH₂ₜ-Het² und/oder COOR⁷ substituiert sein kann, CF₃, C₃-C₈-Cycloalkyl, Ar oder Het²,
- R⁷ und R⁸: jeweils A, C₂-C₆-Alkenyl, C₂-C₆-Alkinyl, C₃-C₈-Cycloalkyl-CₖH₂ₖ- oder C₁-C₆-Alkyl, worin eine CH₂-Gruppe durch O oder S ersetzt ist,
- R⁹ und R¹⁰: jeweils H, A, C₂-C₆-Alkenyl oder C₂-C₆-Alkinyl, Ar, ArCₙH₂ₙ- oder Het²,
- R⁹ auch: -CH₂COOA, -SO₂-A oder -SO₂-Ar,
- R⁹ und R¹⁰: zusammen auch eine Alkylenkette mit 2-5 C-Atomen, die ein- oder mehrfach durch Carbonylsauerstoff, Ar, Het², -CO-Ar, -COOA, -CO-N(A)₂, -CH₂OH, -SO₂-Ar und/oder -NH-CO-A substituiert und/oder durch O oder durch -NR¹⁹- unterbrochen sein kann,
- R¹¹: C₁-C₅-Alkyl, worin auch ein oder mehrere H-Atom(e) durch F ersetzt sein können,
- R¹²: C₃-C₈-Cycloalkyl, CN, COOA, COOH, Ar, Het¹, Het², 1H-5-Tetrazolyl, -CO-NR⁹R¹⁰, -CO-R¹¹, -CO-Ar, -CO-Het², -CO-R¹⁷, -C(=NR¹⁵)-A, -C(=NR¹⁵)-Het², -S(O)ₘ-A, -S(O)ₘ-Ar, -S(O)ₘ-Het², -SO₂-NH-Het² oder -SO₂-OR¹⁸,
- R¹³: COOH, COOA, CONR⁹R¹⁰, CN, NO₂, NHCOR¹⁴, NHSO₂R¹⁴ oder 1H-5-Tetrazolyl,
- R¹⁴: Ar oder Cycloalkyl mit 3-8 C-Atomen,
- R¹⁵: H, OH, CN, R¹⁶, OR¹⁶ oder OAr,
- R¹⁶: A, C₂-C₆-Alkenyl oder C₂-C₆-Alkinyl,
- R¹⁷: -NH-CHR¹⁸-COOH, -NH-CHR¹⁸-COOA, -CH₂S(O)ₘ-Ar, -CH₂-COOA, -CₙH₂ₙ-NO₂, -CₙH₂ₙ-NR⁹R¹⁰ oder -CₙH₂ₙ-NHCOOA,
- R¹⁸: H oder A,
- R¹⁹: H, A, Ar, COOA, Het² oder SO₂-Ar,
- X: fehlt, -NH-CO- oder -CO-NH-,
- Y: O oder S,
- A: C₁-C₆-Alkyl,
- Ar: eine unsubstituierte oder eine durch R¹¹, OH, OR¹¹, COOH, COOA, CN, NO₂, NH₂, NHA, N(A)₂, NHCOR¹¹, NHCOOA, NHSO₂R¹¹, Hal und/oder 1H-5-Tetrazolyl mono- oder disubstituierte Phenylgruppe,
- Het¹: einen fünf- oder sechsgliedrigen gesättigten heterocyclischen Rest mit 1 bis 3 N-, O- und/oder S-Atomen, der einfach durch Carbonylsauerstoff oder =NR¹⁵ und/oder dessen Ring-N-Atom(e) jeweils durch A oder Ar substituiert sein kann (können),
- Het²: einen fünf- oder sechsgliedrigen heteroaromatischen Rest mit 1 bis 3 N-, O- und/oder S-Atomen, der auch mit einem Benzol- oder Pyridinring kondensiert sein kann,
- Hal: F, Cl, Br oder I,
- k: 0, 1, 2, 3 oder 4,
- m: 0, 1 oder 2,
- n: 1, 2, 3, 4, 5 oder 6 und
- t: 1, 2, 3 oder 4 bedeuten,
sowie ihre Salze.

Ähnliche Verbindungen sind aus der EP-A2-0400 974 bekannt.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze bei guter Verträglichkeit sehr wertvolle pharmakologische Eigenschaften besitzen. Insbesondere zeigen sie angiotensin II-antagonistische Eigenschaften und können daher als Arzneimittelwirkstoffe zur Prophylaxe und/oder Therapie von Herz-, Kreislauf- und Gefäßkrankheiten, vor allem zur Behandlung der angiotensin II-abhängigen Hypertension, des Aldosteronismus, der Herzinsuffizienz und des erhöhten Augeninnendrucks sowie von Störungen des Zentralnervensystems eingesetzt werden, ferner von Hypertrophie und Hyperplasie der Blutgefäße und des Herzens, Angina pectoris, Herzinfarkt, Schlaganfall, Restenosen nach Angioplastie oder By-pass-Operationen, Arteriosklerose, Glaukomen, macularer Degeneration, Hyperurikämie, Nierenfunktionsstörungen, z.B. Nierenversagen, Nephropathia diabetica, Retinopathia diabetica, Psoriasis, angiotensin II-vermittelten Störungen in weiblichen Fortpflanzungsorganen, Wahrnehmungsstörungen, z.B. Demenz, Amnesie, Gedächtnisfunktionsstörungen, Angstzuständen, Depression und/oder Epilepsie.

Diese Wirkungen können nach üblichen in vitro- oder in vivo-Methoden ermittelt werden, wie sie z.B. in der US-PS 4 880 804, der US-PS 5 036 048 und der WO 91/14367 beschrieben sind, ferner von A.T. Chiu et al., J. Pharmacol. Exp. Therap. 250, 867-874 (1989), und von P.C. Wong et al., ibid. 252, 719-725 (1990; in vivo, an Ratten).

Gegenstand der Erfindung sind die Verbindungen der Formel I und ihre Salze sowie ein Verfahren zur Herstellung dieser Verbindungen sowie ihrer Salze, dadurch gekennzeichnet, daß man
(a) eine Verbindung der Formel II 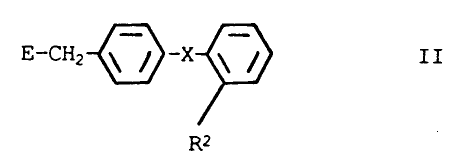 worin
   - E: Cl, Br, I oder eine freie oder reaktionsfähig funktionell abgewandelte OH-Gruppe bedeutet und R² und X die in Anspruch 1 angegebene Bedeutung haben, mit einer Verbindung der Formel III

   H-R III

   worin
   - R: die in Anspruch 1 angegebene Bedeutung hat,
   umsetzt
   oder
(b) eine Verbindung der Formel IV 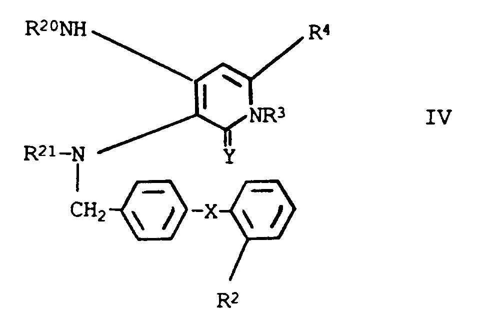 worin
   - R²⁰: R¹-CO oder H und
   - R²¹: H (falls R²⁰ R¹-CO ist) oder R¹-CO (falls R²⁰ H ist)
   bedeuten und
   R¹, R², R³, R⁴, X und Y die in Anspruch 1 angegebenen Bedeutungen haben,
   mit einem cyclisierenden Mittel behandelt,
   oder
(c) zur Herstellung einer Verbindung der Formel I, worin X -NH-CO- oder -CO-NH- bedeutet, eine Verbindung der Formel V 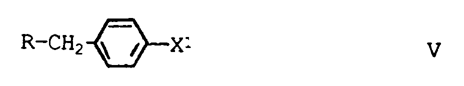 worin
   - X¹: NH₂ oder COOH bedeutet und
   - R: die in Anspruch 1 angegebene Bedeutung hat
   oder ein reaktionsfähiges Derivat dieser Verbindung mit einer Verbindung der Formel VI 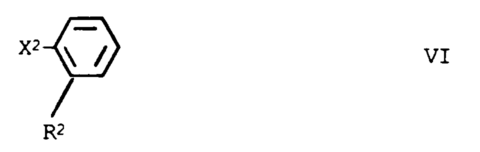 worin
   - X²: COOH (falls X¹ NH₂ ist) oder NH₂ (falls X¹ COOH ist) bedeutet und
   - R²: die in Anspruch 1 angegebene Bedeutung hat,
   oder mit einem reaktionsfähigen Derivat dieser Verbindung umsetzt oder
(d) zur Herstellung einer Verbindung der Formel I, worin R² -C(NH₂)=NOH bedeutet, eine Verbindung, die der Formel I entspricht, aber an Stelle des Restes R² eine CN-Gruppe trägt, mit Hydroxylamin umsetzt oder
(e) zur Herstellung einer Verbindung der Formel I, worin R² -SO₂NH-COOR⁵, -SO₂NH-COR⁵, -SO₂NH-SO₂R⁵ oder -SO₂NH-CONR⁵R⁶ bedeutet, eine Verbindung, die der Formel I entspricht, aber an Stelle des Restes R² eine -SO₂NH₂-Gruppe trägt, mit einer Verbindung der Formel E-COOR⁵, E-COR⁵, E-SO₂R⁵, E-CONR⁵R⁶ oder O=C=NR⁵ umsetzt oder
(f) eine Verbindung der Formel I aus einem ihrer funktionellen Derivate durch Behandeln mit einem solvolysierenden oder hydrogenolysierenden Mittel in Freiheit setzt,
und/oder daß man in einer Verbindung der Formel I einen oder mehrere Rest(e) R, R² und/oder R³, in einen oder mehrere andere Reste R, R² und/oder R³ umwandelt und/oder eine Base oder Säure der Formel I in eines ihrer Salze umwandelt.

Vor- und nachstehend haben die Reste bzw. Parameter R, R¹ bis R²¹, X, Y, A, Ar, Het¹, Het², Hal, k, m, n, t, E, X¹ und X² die bei den Formeln I bis VIII angegebenen Bedeutungen, falls nicht ausdrücklich etwas anderes angegeben ist.

In den vorstehenden Formeln hat A 1-6, vorzugsweise 1, 2, 3 oder 4 C-Atome. A bedeutet vorzugsweise Methyl, weiterhin Ethyl, Propyl, Isopropyl, Butyl, Isobutyl, sek.-Butyl, oder tert.-Butyl, ferner auch Pentyl. 1-, 2- oder 3-Methylbutyl, 1,1-, 1,2- oder 2,2-Dimethylpropyl, 1-Ethylpropyl, Hexyl, 1-, 2-, 3- oder 4-Methylpentyl, 1,1-, 1,2, 1,3-, 2,2-, 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl. Alkenyl steht vorzugsweise für Vinyl, 1- oder 2-Propenyl, 1-Butenyl, ferner 1-Pentenyl oder 1-Hexenyl. Alkinyl steht vorzugsweise für Ethinyl, 1- oder 2-Propinyl, ferner 1-Butinyl, 1-Pentinyl oder 1-Hexinyl. Falls mehrere Reste A, Alkenyl oder Alkinyl in einer Verbindung der Formel I vorhanden sind, so können sie gleich oder voneinander verschieden sein.

Hal bedeutet vorzugsweise F, Cl oder Br, aber auch J.

R ist ein von 3H-Imidazo[4,5-c]pyridin ("3H-IP") abgeleiteter Rest, genauer 2-R¹-4-(thi)oxo-5-R³-6-R⁴-4,5-dihydro-3H-imidazo[4,5-c]pyridin-3-yl.

Ar ist vorzugsweise unsubstituiertes, ferner - wie angegeben - monosubstituiertes Phenyl, im einzelnen bevorzugt Phenyl, o-, m- oder p-Tolyl, o-, m- oder p-Ethylphenyl, o-, m- oder p-Trifluormethylphenyl, o-, m- oder p-Methoxyphenyl, o-, m- oder p-Ethoxyphenyl, o-, m- oder p-(Difluormethoxy)-phenyl, o-, m- oder p-Trifluormethoxyphenyl, o-, m- oder p-Carboxyphenyl, o-, m- oder p-Methoxycarbonylphenyl, o-, m- oder p-Ethoxycarbonylphenyl, o-, m- oder p-Cyanphenyl, o-, m- oder p-Nitrophenyl, o-, m- oder p-Aminophenyl, o-, m- oder p-Methylaminophenyl, o-, m- oder p-Dimethylaminophenyl, o-, m- oder p-Trifluoracetamidophenyl, o-, m- oder p-Methoxycarbonylamino, o-, m- oder p-Ethoxycarbonylamino, o-, m- oder p-Methylsulfonamidophenyl, o-, m- oder p-Trifluormethylsulfonamidophenyl, o-, m- oder p-Fluorphenyl, o-, m- oder p-Chlorphenyl, o-, m- oder p-Bromphenyl, o-, m- oder p-(1H-5-Tetrazolyl)-phenyl, ferner bevorzugt 2,3-, 2,4-, 2,5-, 2,6-, 3,4- oder 3,5-Dimethylphenyl, 2,3-, 2,4-, 2,5-, 2,6-, 3,4- oder 3,5-Dimethoxyphenyl.

Het¹ ist vorzugsweise Tetrahydro-2- oder -3-furyl, Tetrahydro-2- oder -3-thienyl, 1-, 2- oder 3-Pyrrolidinyl, 2-, 3-, 4- oder 5-Oxazolidinyl, 2-, 3-, 4- oder 5-Thiazolidinyl, 1-, 2-, 3-, 4- oder 5-Imdazolidinyl, 2-, 3- oder 4-Tetrahydropyranyl, 2-, 3- oder 4-Tetrahydrothiopyranyl, 1-, 2-, 3- oder 4-Piperidinyl, 2-, 3- oder 4-Morpholinyl, 1,- 2- oder 3-Piperazinyl, 1-Methyl-2- oder -3-pyrrolidinyl, 1-Methyl-2-, -3- oder -4-piperidinyl, 4-Methyl-2- oder -3-morpholinyl, 1-Methyl-2-, -3- oder -4-piperazinyl, 1-Phenyl-2- oder -3-pyrrolidinyl, 1-Phenyl-2-, -3- oder -4-piperidinyl, -4-Phenyl-2- oder -3-morpholinyl, 1-Phenyl-2-, -3- oder 4-piperazinyl, 2-Oxo-3-, -4- oder -5-oxazolidinyl, 2-Oxo-3-, -4- oder -5-thiazolidinyl, 2-Oxo-1-, -3-, -4- oder -5-imidazolidinyl, 2,4-Dioxo-1-, -3- oder -5-imidazolidinyl, 2-Oxo-3-phenyl-4- oder -5-oxazolidinyl, 2-Oxo-3-o-, -m- oder -p-tolyl-4- oder -5-oxazolidinyl, 2-Hydroxyimino-3-, -4- oder -5-oxazolidinyl, 2-Methoxyimino-3-, -4- oder -5-oxazolidinyl, 2-Hydroxyimino-4-oxo-3- oder -5-oxazolidinyl, 2-Methoxyimino-4-oxo-3- oder -5-oxazolidinyl.

Het² ist vorzugsweise 2- oder 3-Furyl, 2- oder 3-Thienyl, 1-, 2- oder 3-Pyrrolyl, 1-, 2-, 4- oder 5-Imidazolyl, 1-, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4- oder -5-yl, 1,2,4-Triazol-1-, -3- oder -5-yl, 1,2,3-Oxadiazol-4- oder -5-yl, 1,2,4-Oxadiazol-3- oder -5-yl, 1,3,4-Thiadiazol-2- oder -5-yl, 1,2,4-Thiadiazol-3- oder -4-yl, 2,1,5-Thiadiazol-3- oder -4-yl, 3- oder 4-Pyridazinyl, Pyrazinyl, 2-, 3-, 4-, 5-, 6- oder 7-Benzofuryl, 2-, 3-, 4-, 5-, 6- oder 7-Benzothienyl, 1-, 2-, 3-, 4-, 5-, 6- oder 7-Indolyl, 1-, 2-, 3-, 4-, 5-, 6- oder 7-Isoindolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7-Benzisoxazolyl, 2-, 4-, 5-, 6- oder 7-Benzthiazolyl, 2-, 4-, 5-, 6- oder 7-Benzisothiazolyl, 4-, 5-, 6- oder 7-Benz-2-1,3-oxadiazolyl, 2-, 3-, 4-, 5-, 6-, 7- oder 8-Chinolyl, 1-, 3-, 4-, 5-, 6-, 7- oder 8-Isochinolyl, 3-, 4-, 5-, 6-, 7- oder 8-Cinnolyl, 2-, 4-, 5-, 6-, 7- oder 8-Chinazolyl, 1H-1-, -2-, -5-, -6- oder -7-Imidazo[4,5-b]pyridyl, 3H-2-, -3-, -5-, -6- oder -7-Imidazo[4,5-b]pyridyl, 1H-1-, -2-, -4-, -6- oder -7-Imidazo[4,5-c]-pyridyl, 3H-2-, -3-, -4-, -6- oder -7-Imidazo[4,5-c]pyridyl.

In den Begriff "Het²" eingeschlossen sind auch die homologen Reste, in denen der heteroaromatische Ring durch eine oder mehrere, vorzugsweise 1 oder 2, A-Gruppen, vorzugsweise Methyl- und/oder Ethylgruppen substituiert ist, z.B. 3-, 4- oder 5-Methyl-2-furyl, 2-, 4- oder 5-Methyl-3-furyl, 2,4-Dimethyl-3-furyl, 3-, 4- oder 5-Methyl-2-thienyl, 3-Methyl-5-tert.-butyl-2-thienyl, 2-, 4- oder 5-Methyl-3-thienyl, 2- oder 3-Methyl-1-pyrrolyl, 1-, 3-, 4- oder 5-Methyl-2-pyrrolyl, 3,5-Dimethyl-4-ethyl-2-pyrrolyl, 2-, 4- oder 5-Methyl-1-imidazolyl, 4-Methyl-5-pyrazolyl, 4- oder 5-Methyl-3-isooxazolyl, 3- oder 5-Methyl-4-isoxazolyl, 3- oder 4-Methyl-5-isoxazolyl, 3,4-Dimethyl-5-isoxazolyl, 4- oder 5-Methyl-2-thiazolyl, 4- oder 5-Ethyl-2-thiazolyl, 2- oder 5-Methyl-4-thiazolyl, 2- oder 4-Methyl-5-thiazolyl, 2,4-Dimethyl-5-thiazolyl, 3-, 4-, 5- oder 6-Methyl-2-pyridyl, 2-, 4-, 5- oder 6-Methyl-3-pyridyl, 2- oder 3-Methyl-4-pyridyl, 4-Methyl-2-pyrimidinyl, 4,5-Dimethyl-2-pyrimidinyl, 2-, 5- oder 6-Methyl-4-pyrimidinyl, 2,6-Dimethyl-4-pyrimidinyl, 3-, 4-, 5-, 6- oder 7-Methyl-2-benzofuryl, 2-Ethyl-3-benzofuryl, 3-, 4-, 5-, 6- oder 7-Methyl-2-benzothienyl, 3-Ethyl-2-benzothienyl, 1-, 2-, 4-, 5-, 6- oder 7-Methyl-3-indolyl, 1-Methyl-5- oder 6-benzimidazolyl, 1-Ethyl-5- oder 6-benzimidazolyl.

Die Gruppen -CₖH₂ₖ-, -CₙH₂ₙ- und -CₜH₂ₜ- sind vorzugsweise geradkettig und stehen somit bevorzugt für -(CH₂)ₙ-, -(CH₂)ₖ- und -(CH₂)ₜ-, insbesondere für -CH₂-, ferner für -CH₂CH₂-, -(CH₂)₃-, -(CH₂)₄-, -(CH₂)₅- oder -(CH₂)₆-, aber auch z.B. für -CH(CH₃)-, -CH₂-CH(CH₃)- oder -C(CH₃)₂-. Der Parameter k kann bevorzugt auch 0 sein, so daß die Gruppe -CₖH₂ₖ- fehlt.

Der Rest R¹ ist vorzugsweise geradkettig und steht bevorzugt für A, insbesondere Ethyl, Propyl oder Butyl, ferner Methyl, Pentyl oder Hexyl, sowie für Cycloalkyl mit 3-7 C-Atomen, insbesondere Cyclopropyl, ferner Cyclobutyl, Cyclopentyl, Cyclohexyl, Cycloheptyl, weiterhin insbesondere für Alkenyl mit vorzugsweise 3-6 C-Atomen, vor allem Allyl oder 1-Propenyl, ferner 1-Butenyl, 1-Pentenyl oder 1-Hexenyl; für Alkinyl mit vorzugsweise 3-6 C-Atomen, vor allem Propargyl oder 1-Propinyl, ferner 1-Butinyl, 1-Pentinyl oder 1-Hexinyl; für Cycloalkylalkyl mit vorzugsweise 4-8 C-Atomen, vor allem Cyclopropylmethyl, 1- oder 2-Cyclopropylethyl, ferner Cyclobutylmethyl, Cyclopentylmethyl, Cyclohexylmethyl; für Alkoxy mit vorzugsweise 1-4 C-Atomen wie Methoxy, Ethoxy, Propoxy, Butoxy, Isobutoxy; für Alkoxyalkyl mit vorzugsweise 2-5 C-Atomen wie Methoxymethyl, Ethoxymethyl, Propoxymethyl, 2-Methoxyethyl, 3-Methoxypropyl, 2-Ethoxyethyl; für Alkylthio mit vorzugsweise 1-4 C-Atomen wie Methylthio, Ethylthio, Propylthio, Butylthio, Isobutylthio; für Alkylthioalkyl mit vorzugsweise 2-5 C-Atomen wie Methylthiomethyl, Ethylthiomethyl, Propylthiomethyl, 2-Methylthio-ethyl, 3-Methylthiopropyl, 2-Ethylthio-ethyl.

Der Rest R² ist vorzugsweise -SO₂NH-COOR⁵, insbesondere -SO₂NH-COOA; -SO₂NH-CO-R⁵, insbesondere -SO₂NH-COAr wie -SO₂NH-COC₆H₅; -SO₂NH-CONR⁵R⁶, insbesondere -SO₂NH-CO-NHAr wie -SO₂NH-CO-NHC₆H₅ oder -SO₂NH-CO-NHCH₂Het² wie -SO₂NH-CO-NHCH₂-(2-pyridyl). Der Rest R² ist ferner vorzugsweise 4,5-Dihydro-5-oxo-1,2,4-oxadiazol-3-yl, ferner bevorzugt 4,5-Dihydro-5-thioxo-1,2,4-oxdiazol-3-yl, 2-Oxo-3H-1,2,3,5-oxathiadiazol-4-yl, 2,2-Dioxo-3H-1,2,3,5-oxathiadiazol-4-yl oder 4,5-Dihydro-5-oxo-1,2,4-thiadiazol-3-yl.

Der Rest R³ steht bevorzugt für H; R¹¹ insbesondere CH₃, CF₃, C₂H₅, C₂F₅, CH₂CF₃, CH₂CH₂CF₃; Ar-C₂-C₆-alkenyl, z.B. Cinnamyl; im "Alkenyl"-Teil durch COOA substituiertes Ar-C₂-C₆-alkenyl, z.B. 3-Ethoxycarbonyl-2-phenyl-2-propen-1-yl; -CₙH₂ₙ-R¹² (im einzelnen bevorzugt für -CH₂-R¹²), insbesondere für -CₙH₂ₙ-C₃-C₈-cycloalkyl (wie Cyclopropylmethyl), Cyclobutylmethyl, Cyclopentylmethyl, Cyclohexylmethyl, -CₙH₂ₙ-CN (wie Cyanmethyl, 2-Cyanethyl, 3-Cyanpropyl), -CₙH₂ₙ-COOA (wie Methoxycarbonylmethyl, Ethoxycarbonylmethyl, 2-Methoxycarbonylethyl, 2-Ethoxycarbonyl-ethyl), -CₙH₂ₙ-COOH (wie Carboxymethyl, 2-Carboxyethyl, 3-Carboxypropyl), -CₙH₂ₙ-Ar (wie Benzyl, 1- oder 2-Phenylethyl, 1-, 2- oder 3-Phenylpropyl, 1-, 2-, 3- oder 4-Phenylbutyl, o-, m- oder p-Fluorbenzyl, (bevorzugt) o-, m- oder p-Chlorbenzyl, o-, m- oder p-Brombenzyl, o-, m- oder p-Methylbenzyl, o-, m- oder p-Trifluormethylbenzyl, o-, m- oder p-Methoxycarbonylbenzyl, o-, m- oder p-Ethoxycarbonylbenzyl, o-, m- oder p-Cyanbenzyl, o-, m- oder p-Carboxybenzyl, o-, m- oder p-Nitrobenzyl, o-, m- oder p-Aminobenzyl, o-, m- oder p-Trifluoracetamidobenzyl, o-, m- oder p-Trifluormethylsulfonamidobenzyl, o-, m- oder p-(1H-5-Tetrazolyl)-benzyl, 2-Chlor-6-nitrobenzyl); -CₙH₂ₙ-Het¹ (bevorzugt -CH₂-Het¹ wie -CH₂-(2-oxo-3-Ar-5-oxazolidinyl), z.B. 2-Oxo-3-m-tolyl-5-oxazolidinylmethyl); -CₙH₂ₙ-Het² (bevorzugt -CH₂-Het² wie 2- oder 3-Furylmethyl, 2- oder 3-Thienylmethyl, 5-Isoxazolmethyl, 5-Methyl-3-isoxazolylmethyl, 2-, 3- oder 4-Pyridylmethyl, Pyrazinylmethyl, 2-, 4-, 5- oder 6-Pyrimidinylmethyl, 3- oder 4-Pyridazinylmethyl, 2-, 3-, 4-, 5-, 6- oder 7-Benzofurylmethyl, 2-, 3-, 4-, 5-, 6- oder 7-Benzothienylmethyl, 2-, 3-, 4-, 5-, 6- oder 7-Indolylmethyl); -CₙH₂ₙ-(1H-5-Tetrazolyl) (wie 1H-5-Tetrazolyl-methyl, 2-(1H-5-Tetrazolyl)-ethyl, 3-(1H-5-Tetrazolyl)-propyl); -CₙH₂ₙ-CONR⁹R¹⁰ (worin n bevorzugt 1 oder 2, R⁹ bevorzugt H oder A und R¹⁰ bevorzugt H, A, Ar, ArCₙH₂ₙ oder Het² bedeuten, wie Carbamoylmethyl, 2-Carbamoylethyl, N-Methyl-carbamoylmethyl, 2-N-Methylcarbamoylethyl, N-Ethyl-carbmoylmethyl, N-Propylcarbamoylmethyl, N-Isopropyl-carbamoylmethyl, N-Butyl-carbamoylmethyl, N-Isobutyl-carbamoylmethyl, N-sek.-Butyl-carbamoylmethyl, N-tert.-Butyl-carbamoylmethyl, N,N-Dimethyl-carbamoylmethyl, 2-N,N-Dimethyl-carbamoylethyl, N-Methyl-N-ethylcarbamoylmethyl, N,N-Diethyl-carbamoylmethyl, N,N-Dipropylcarbamoylmethyl, N,N-Diisopropyl-carbamoylmethyl, N,N-Dibutyl-carbamoylmethyl; ferner z.B. Pyrrolidinocarbonylmethyl, Piperidinocarbonylmethyl, Morpholinocarbonylmethyl); -CₙH₂ₙ-CO-NHAr, z.B. N-Phenyl-carbamoylmethyl, 2-N-Phenyl-carbamoylethyl, N-o-, -m- oder -p-Tolyl-carbamoylmethyl, N-o-, m- oder -p-Trifluormethylphenyl-carbamoylmethyl, N-o-, -m- oder -p-Carboxyphenyl-carbamoylmethyl, N-o-, -m- oder -p-Ethoxycarbonylphenyl-carbamoylmethyl, N-o-, -m- oder p-Fluorphenyl-carbamoylmethyl, N-o-, -m- oder-p-Chlorphenyl-carbamoylmethyl, N-(2,3, N-(2,4-, N-(2,5-, N-(2,6-, N-(3,4- oder N-(3,5-Dimethylphenyl)-carbamoylmethyl, 2-N-(2,3-, 2-N-(2,4-, 2-N-(2,5-, 2-N-(2,6-, 2-N-(3,4- oder 2-N-(3,5-Dimethylphenyl)-carbamoylethyl; -CₙH₂ₙ-CO-NH-Het², z.B. N-(2-, N-(3- oder N-(4-Pyridyl)-carbamoylmethyl, 2-N-(2-Pyridyl)-carbamoylethyl, N- (2- oder N-(3-Thienyl)-carbamoylmethyl; -CₙH₂ₙ-CO-NAAr, z.B. N-Methyl-N-phenyl-carbamoylmethyl, 2-N-Methyl-N-phenyl-carbamoylethyl, N-Ethyl-N-phenyl-carbamoylmethyl; -CₙH₂ₙ-CO-NA(CₙH₂ₙ-Ar), z.B. N-Methyl-N-benzylcarbamoylmethyl, N-Methyl-N-(2-phenylethyl)-carbamoylmethyl, N-Methyl-N-(1,1-dimethyl-2-phenylethyl)-carbamoylmethyl, 2-N-Methyl-N-(1,1-dimethyl-2-phenylethyl)-carbamoylethyl; -CₙH₂ₙ-CO-N(Ar)₂, z.B. N,N-Diphenylcarbamoylmethyl ; -CₙH₂ₙ-CO-R¹¹ (bevorzugt -CH₂-CO-R¹¹ wie 2-Oxopropyl, 2-Oxobutyl, 3-Methyl-2-oxobutyl, 3,3-Dimethyl-2-oxobutyl, 3,3,3-Trifluor-2-oxopropyl, 3,3,4,4,4-Pentafluor-2-oxobutyl); -CₙH₂ₙ-CO-Ar (bevorzugt -CH₂-CO-Ar wie Phenacyl (= 2-Oxo-2-phenyl-ethyl), o-, m- oder p-Methylphenacyl, o-, m- oder p-Ethylphenacyl, o-, m- oder p-Trifluormethylphenacyl, o-, m- oder p-Methoxyphenacyl, o-, m- oder p-Ethoxyphenacyl, o-, m- oder p-(Difluormethoxy)-phenacyl, o-, m- oder p-(Trifluormethoxy)-phenacyl, o-, m- oder p-Carboxyphenacyl, o-, m- oder p-Methoxycarbonylphenacyl, o-, m- oder p-Ethoxycarbonylphenacyl, o-, m- oder p-Cyanphenacyl, o-, m- oder p-Nitrophenacyl, o-, m- oder p-Aminophenacyl, o-, m- oder p-Acetamidophenacyl, o-, m- oder p-Trifluoracetamidophenacyl, o-, m- oder p-Methylsulfonamidophenacyl, o-, m- oder p-Trifluormethylsulfonamidophenacyl, o-, m- oder p-(1H-5-Tetrazolyl)phenacyl); -CₙH₂ₙ-CO-Het² (bevorzugt -CH₂-CO-Het² wie 2-Furoylmethyl, 2-Thenoylmethyl, Picolinoylmethyl, Nicotinoylmethyl, Isonicotinoylmethyl, Pyrazin-carbonylmethyl, 2-, 4-, 5- oder 6-Pyrimidincarbonylmethyl, 3- oder 4-Pyridazincarbonylmethyl, Benzofuran-2-, -3-, -4-, -5-, -6- oder -7-carbonylmethyl, Benzothiophen-2-, -3-, -4-, -5-, -6- oder -7-carbonylmethyl, Indol-2-, -3-, -4-, -5-, -6- oder -7-carbonylmethyl); -CₙH₂ₙ-CO-CH₂-NO₂, z.B. 3-Nitro-2-oxopropyl, 4-Nitro-3-oxopropyl; -(CH₂)ₜ-CO-CₙH₂ₙ-NH-COOA, z.B. 4-BOC-amino-2-oxobutyl, 5-BOC-amino-2-oxopentyl, 6-BOC-amino-2-oxohexyl; -CₙH₂ₙ-CO-CₙH₂ₙ-NH₂, z.B. 3-Amino-2-oxopropyl,4-Amino-2-oxobutyl, 5-Amino-2-oxopentyl, 6-Amino-2-oxohexyl, 4-Amino-3-oxobutyl; -CₙH₂ₙ-CO-NH-SO₂Ar, z.B. N-Phenylsulfonylcarbamoylmethyl; -CₙH₂ₙ-C(=NR¹⁵)-A (bevorzugt -CH₂-C(=NR¹⁵)-A wie -CH₂-(=NOH)-CH₃, -CH₂-C(=NOCH₃)-C(CH₃)₃; -CₙH₂ₙ-S-A, z.B. Methylthiomethyl; -CₙH₂ₙ-SO-A, z.B. Methylsulfinylmethyl; -CₙH₂ₙ-SO₂-A, z.B. Methylsulfonylmethyl; -CₙH₂ₙ-S-Ar, z.B. Phenylthio-methyl; -CₙH₂ₙ-SO-Ar, z.B. Phenylsulfinyl-methyl; -CₙH₂ₙ-SO₂-Ar, z.B. Phenylsulfonyl-methyl; -CₙH₂ₙ-S-Het², z.B. (2-Thienyl)-thiomethyl; -CₙH₂ₙ-SO-Het², z.B. (2-Pyridyl)-sulfinylmethyl; -CₙH₂ₙ-SO₂-Het², z.B. (2-; (3- oder (4-Pyridyl)-sulfonylmethyl; -CH(COOA)-Ar, z.B. α-Methoxycarbonylbenzyl, α-Ethoxycarbonylbenzyl, α-Isopropoxycarbonylbenzyl. Weiterhin kann der Rest R³ auch 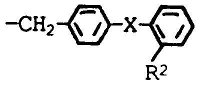
bedeuten.

Der Rest R⁵ ist vorzugsweise A, insbesondere Methyl, Ethyl, Propyl oder Butyl; Ar, insbesondere Phenyl; Het²-alkyl, insbesondere 2-, 3- oder 4-Pyridyl-methyl; oder Cycloalkyl, insbesondere Cyclopropyl.

Der Rest R⁶ ist vorzugsweise H, ferner A.

Die Reste R⁷ und R⁸ sind vorzugsweise jeweils A.

Die Reste R⁹ und R¹⁰ stehen vorzugsweise für H oder A, R⁹ steht zusätzlich bevorzugt für Ar, Ar-CₙH₂ₙ oder Het².

Weitere bevorzugte Gruppen -NR⁹R¹⁰ sind diejenigen, in denen R⁹ und R¹⁰ zusammen eine Alkylenkette mit 2-5 C-Atomen bedeuten, die wie angegeben substituiert und/oder durch O oder durch -NR¹⁹- unterbrochen sein kann. Besonders bevorzugte Gruppen -NR⁹R¹⁰ dieser Art sind z.B. Aziridino, Pyrrolidino, Piperidino, Morpholino, Piperazino, 2-Oxo-pyrrolidino, 2-Alkoxycarbonyl-pyrrolidino (worin die Alkoxygruppe 1-4 C-Atome enthält) wie 2-Methoxycarbonyl-pyrrolidino oder 2-Ethoxycarbonyl-pyrrolidino, 2- oder 3-Alkanoylaminopyrrolidino wie 2- oder 3-Acetamido-pyrrolidino, 2-, 3- oder insbesondere 4-Oxo-piperidino, 2-, 3- oder insbesondere 4-Ar-piperidino wie 2-, 3- oder 4-Phenyl-piperidino, 4-o-, 4-m- oder 4-p-Methoxyphenyl-piperidino, 4-o, 4-m- oder 4-p-Nitrophenyl-piperidino, 4-o-, 4-m- oder 4-p-Chlorphenyl-piperidino, 3-Hydroxmethyl-4-p-chlorphenyl-piperino, 2-, 3- oder 4-(2-Thienyl)-piperidino, 2-, 3- oder 4-N,N-Dimethylcarbamoyl-piperidino, 2-, 3- oder 4-N,N-Diethylcarbamoyl-piperidino, 2-, 3- oder 4-Benzoyl-piperidino, 2-, 3- oder 4-p-Methoxybenzoyl-piperidino, 4-Methylpiperazino, 4-Phenyl-piperazino, 4-o-, 4-m- oder 4-p-Methoxyphenyl-piperazino, 4-o-, 4-m- oder 4-p-Nitrophenyl-piperazino, 4-o-, 4-m- oder 4-p-Chlorphenyl-piperazino, 4-(2-Pyrimidinyl)-piperazino, 4-Methoxycarbonyl-piperazino, 4-Ethoxycarbonyl-piperazino, 4-BOC-piperazino, 4-Phenylsulfonyl-piperazino, 4-p-Tolylsulfonyl-piperazino, 4-o-, 4-m- oder 4-p-Fluorphenylsulfonyl-piperazino.

Der Rest R¹¹ enthält bevorzugt 1, 2 oder 3-C-Atome und bedeutet vorzugsweise Methyl, Ethyl, Trifluormethyl, Pentafluorethyl, 2,2,2-Trifluorethyl oder 3,3,3-Trifluorpropyl. Falls eine Verbindung der Formel I zwei Reste R⁵ enthält, so können diese gleich oder voneinander verschieden sein.

R¹² ist vorzugsweise Ar, -COOA, -COOH oder -CO-NR⁹R¹⁰, ferner bevorzugt -CO-R¹¹, -CO-Ar, -CO-R¹⁷ oder -C(=NR¹⁵)-A.
R¹³ ist vorzugsweise COOH oder COOA.
R¹⁴ ist vorzugsweise Ar, inbesondere Phenyl.
R¹⁵ ist vorzugsweise OH oder OR¹⁶, insbesondere OA.
R¹⁶ ist vorzugsweise A.
R¹⁷ ist vorzugsweise -CₙH₂ₙ-NO₂ oder -CₙH₂ₙ-NR⁹R¹⁰, insbesondere -CₙH₂ₙ-NH₂.
R¹⁸ ist vorzugsweise H, ferner A mit 1-4 C-Atomen.
R¹⁹ ist vorzugsweise H oder A.

Der Parameter k ist vorzugsweise 0 oder 1. Der Parameter m ist vorzugsweise 0 oder 2. Der Parameter n ist vorzugsweise 1, ferner bevorzugt 2, 3 oder 4.

Der Rest X fehlt vorzugsweise oder bedeutet -NH-CO- oder -CO-NH-.

Der Rest Y ist vorzugsweise O, aber auch S.

Die Verbindungen der Formel I können ein odere mehrere chirale Zentren besitzen und daher in verschiedenen - optisch-aktiven oder optisch-inaktiven - Formen vorkommen. Die Formel I umschließt alle diese Formen.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln Ia bis Ic ausgedrückt werden, die der Formel I entsprechen und worin die nicht näher bezeichneten Reste die bei Formel I angegebenen Bedeutungen haben, worin jedoch:
- in Ia: X fehlt;
- in Ib: X -NH-CO- bedeutet;
- in Ic: X -CO-NH- bedeutet.

Verbindungen der Formel Ia sind besonders bevorzugt.

Weiterhin sind bevorzugt:
Verbindungen der Formeln Id sowie Iad bis Icd, die den Verbindungen der Formeln I sowie Ia bis Ic entsprechen, worin jedoch zusätzlich Y ein O-Atom bedeutet;
Verbindungen der Formeln Ie, Iae bis Ide, sowie Iade bis Icde, die den Formeln I, Ia bis Id sowie Iad bis Icd entsprechen, worin jedoch zusätzlich R⁴ H bedeutet;
Verbindungen der Formeln If, Iaf bis Ief, Iaef bis Idef sowie Iadef bis Icdef, die den Formeln I, Ia bis Ie, Iae bis Ide sowie Iade bis Icde entsprechen, worin jedoch zusätzlich R²
(a) -SO₂NH-COOA,
(b) -SO₂-NH-CO-Ar,
(c) -SO₂-NH-CO-Cyclopropyl, oder
(d) 4,5-Dihydro-5-oxo-1,2,4-oxadiazol-3-yl
bedeutet.

Unter diesen sind diejenigen Verbindungen bevorzugt, worin R¹ A oder Alkenyl mit jeweils 3-6 C-Atomen oder Cyclopropyl bedeutet.

Weitere bevorzugte Gruppen von Verbindungen entsprechen der Formel I sowie den anderen vorstehend genannten Formeln, worin jedoch der Rest R³ folgende Bedeutungen hat:
(a) alkenyl-Ar mit 2-6 C-Atomen im "alkenyl"-Teil,
(b) -CₙH₂ₙ-R¹²,
(c) -CₙH₂ₙ-Ar,
(d) -CₙH₂ₙ-CO-NR⁹R¹⁰,
(e) -CH₂-CO-NR⁹R¹⁰, worin R⁹ und R¹⁰ jeweils H, A oder Phenyl bedeuten,
(f) -CH₂-CO-NR⁹R¹⁰, worin R⁹ und R¹⁰ zusammen eine Alkylenkette mit 2-5 C-Atomen bedeuten, die ein- oder mehrfach durch Carbonylsauerstoff, Ar, Het², -CO-Ar, -COOA, -CO-N(A)₂, -CH₂OH, -SO₂-Ar und/oder -NH-CO-A substituiert und/oder durch O oder durch -NR¹⁹- unterbrochen sein kann,
(g) -CH₂-CO-NR⁹R¹⁰, worin -NR⁹R¹⁰ Pyrrolidino, Piperidino oder Morpholino bedeutet,
(h) H,
(i) A,
(j) -CH₂Ar,
(k) -CH₂COOH,
(l) -CH₂COOA,
(m) -CH₂-CO-Ar,
(n) -CH₂-thienyl,
(o) Cinnamyl,
(p) -CH(COOA)-Ar,
(q) -CH₂-S(O)ₘ-Ar,
(r) -CH₂-S-Ar,
(s) -CH₂-SO₂Ar.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart; insbesondere aber in der EP-A2-O 430 709 und in der US-PS 4 880 804) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe, insbesondere diejenigen der Formel IV, können, falls erwünscht, auch in situ gebildet werden, so daß man sie aus dem Reaktionsgemisch nicht isoliert, sondern sofort weiter zu den Verbindungen der Formel I umsetzt.
(a) Die Verbindungen der Formel I können vorzugsweise erhalten werden, indem man Verbindungen der Formel II mit Verbindungen der Formel III umsetzt. Besonders die Biphenylderivate der Formel I (worin X fehlt) sind auf diesem Wege gut erhältlich.
   In den Verbindungen der Formel II bedeutet E vorzugsweise Cl, Br, I oder eine reaktionsfähig funktionell abgewandelte OH-Gruppe wie Alkylsulfonyloxy mit 1-6 C-Atomen (bevorzugt Methylsulfonyloxy) oder Arylsulfonyloxy mit 6-10 C-Atomen (bevorzugt Phenyl- oder p-Tolylsulfonyloxy).
   Die Umsetzung von II mit III erfolgt zweckmäßig, indem man zunächst III durch Behandeln mit einer Base in ein Salz umwandelt, z.B. mit einem Alkalimetallalkoholat wie CH₃ONa oder K-tert.-Butylat in einem Alkohol wie Methanol oder tert.-Butanol oder mit einem Alkalimetallcarbonat wie K₂CO₃ oder mit einem Alkalimetallhydrid wie NaH oder einem Alkalimetallalkoholat in Dimethylformamid (DMF), und dieses dann in einem inerten Lösungsmittel, z.B. einem Amid wie DMF, N-Methylpyrrolidon oder Dimethylacetamid oder einem Sulfoxid wie Dimethylsulfoxid (DMSO), mit II umsetzt, zweckmäßig bei Temperaturen zwischen -20 und 100°, vorzugsweise zwischen 10 und 30°. Als Basen eignen sich auch Alkalimetall-hydrogencarbonate wie NaHCO₃ oder KHCO₃.
(b) Die Verbindungen der Formel I sind weiterhin durch Cyclisierung von Verbindungen der Formel IV erhältlich. Diese Cyclisierung gelingt zweckmäßig durch Erhitzen mit Polyphosphorsäure, Essigsäure oder Diglyme auf Temperaturen zwischen etwa 80 und 180°, vorzugsweise zwischen 120 und 160°.
(c) Säureamide der Formel I (X = -NH-CO- oder -CO-NH-) sind ferner erhältlich durch Umsetzung von Verbindungen der Formel V (oder ihrer reaktionsfähigen Derivate) mit Verbindungen der Formel VI (oder ihrer reaktionsfähigen Derivate).
   Als reaktionsfähige Derivate der Carbonsäuren der Formeln V und VI (X¹ bzw. X² = COOH) eignen sich vorteilhaft die entsprechenden Chloride, Bromide oder Anhydride. Die Umsetzung erfolgt zweckmäßig in Gegenwart eines inerten Lösungsmittel, z.B. eines halogenierten Kohlenwasserstoffs wie Dichlormethan, Chloroform, Trichlorethen oder 1,2-Dichlorethan oder eines Ethers wie Tetrahydrofuran (THF) oder Dioxan, bei Temperaturen zwischen 0 und 150°, vorzugsweise zwischen 20 und 80°. Setzt man Säurehalogenide um, so empfiehlt sich der Zusatz einer Base, z.B. eines tertiären Amins wie Triethylamin, Pyridin oder 4-Dimethylaminopyridin.
(d) Umsetzung eines Nitrils, das der Formel I entspricht, aber an Stelle des Restes R² eine CN-Gruppe enthält, mit Hydroxylamin liefert das entsprechende Carboxamid-oxim I, R² = -C(NH₂)=NOH. Bei dieser Reaktion arbeitet man zweckmäßig in einem inerten Lösungsmittel wie THF bei Temperaturen zwischen 20 und 100°.
(e) Verbindungen der Formel I, worin R² -SO₂NH-COOR⁵, -SO₂NH-COR⁵, -SO₂NH-SO₂R⁵ oder -SO₂NH-CONR⁵R⁶ bedeutet, können durch N-Acylierung von Verbindungen erhalten werden, die der Formel I entsprechen, aber an Stelle eines Restes R² eine -SO₂NH₂-Gruppe enthalten. Als Acylierungsmittel eignen sich z.B. Verbindungen der Formeln E-COOR⁵, z.B. Chlorameisensäure-methyl- und -ethylester; E-COR⁵, z.B. Acetylchlorid, Cyclopropancarbonylchlorid, oder Benzoylchlorid; E-SO₂R⁵, z.B. Methansulfonylchlorid, p-Toluolsulfonylchlorid; E-CO-NR⁵R⁶, z.B. Diphenylcarbamoylchlorid; O=C=NR⁵, z.B. Phenylisocyanat.
   Die Umsetzung wird in der Regel in Gegenwart einer Base, vorzugsweise eines tertiären Amins, z.B. Triethylamin, Pyridin, 4-Dimethylaminopyridin vorgenommen, zweckmäßig bei Temperaturen zwischen 0 und 100°. Ein Überschuß des Amins kann auch als Lösungsmittel dienen. Die Reaktion mit Isocyanaten der Formel O=C=NR⁵ zu den entsprechenden Sulfonylharnstoffen wird bevorzugt bei Temperaturen zwischen 50 und 100° vorgenommen, wobei ein Überschuß des Isocyanats als Lösungsmittel dient.
(f) Weiterhin kann man eine Verbindung der Formel I durch Solvolyse (z.B. Hydrolyse) oder Hydrogenolyse aus einem ihrer funktionellen Derivate in Freiheit setzen.
   So kann man Carbonsäuren der Formel I, die (mindestens) eine COOH-Gruppe enthalten, erhalten durch Verseifung entsprechender Alkylester, z.B. mit NaOH oder KOH in wässeriger Lösung mit oder ohne Zusatz eines inerten organischen Lösungsmittels wie Methanol, Ethanol, THF oder Dioxan bei Temperaturen zwischen 0 und 100°, oder durch Hydrogenolyse entsprechender Benzylester, z.B. an Pd-Kohle bei Drucken zwischen 1 und 200 bar und bei Temperaturen zwischen 0 und 100° in einem der angegebenen inerten Lösungsmittel.
   Es ist auch möglich, eine Verbindung, die der Formel I entspricht, aber an Stelle des Restes R² eine 5-Trichlormethyl-1,2,4-oxadiazol-3-yl-Gruppe enthält (erhältlich durch Reaktion eines Carboxamid-oxims der Formel I, R² = -C(NH₂)=NOH, mit Trichloressigsäure-anhydrid) alkalisch zu spalten, z.B. mit NaOH in Wasser/Dioxan bei 0-10°, wobei man die entsprechende Verbindung I, R² = 4,5-Dihydro-5-oxo-1,2,4-oxadiazol-3-yl, erhält.

Die Ausgangsstoffe, insbesondere diejenigen der Formeln II und VI, sind teilweise bekannt. Falls sie nicht bekannt sind, können sie nach bekannten Methoden in Analogie zu bekannten Stoffen hergestellt werden.

Verbindungen der Formel III (Y = O) sind z.B. erhältlich durch Reaktion von Carbonsäuren der Formel R¹-COOH mit Verbindungen der Formel VII 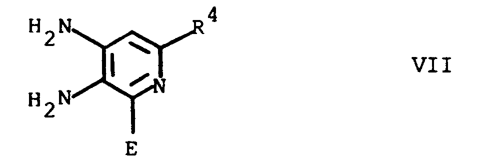
in Gegenwart von Polyphosphorsäure; dabei wird die Gruppe (vorzugsweise Cl) hydrolysiert, und es entstehen zunächst Verbindungen entsprechend Formel III, aber mit R³ = H, die anschließend mit Verbindungen der Formel E-R³ (worin R³ von H verschieden ist) umgesetzt werden können.

Verbindungen der Formel IV sind z.B. erhältlich durch Umsetzung von Verbindungen der Formel VIII 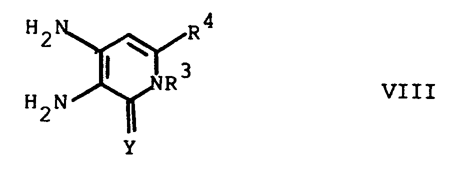
worin jedoch die eine Aminogruppe durch eine Aminoschutzgruppe (z.B. Benzyl, A-O-CO- oder Benzyloxycarbonyl) geschützt ist, mit Verbindungen der Formel II sowie nachfolgende Abspaltung der Schutzgruppe und Reaktion mit Säuren der Formel R¹-COOH oder deren funktionellen Derivaten; sie werden in der Regel nicht isoliert, sondern entstehen in situ bei der letztgenannten Umsetzung.

Verbindungen der Formel V können durch Reaktion von III mit Benzylchloriden der Formel Cl-CH₂-p-C₆H₄-X³ (worin X³ eine geschützte NH₂- oder COOH-Gruppe bedeutet) und nachfolgende Abspaltung der Schutzgruppe hergestellt werden.

Es ist ferner möglich, eine Verbindung der Formel I in eine andere Verbindung der Formel I umzuwandeln, indem man einen oder mehrere der Reste R und/oder R² in andere Reste R und/oder R² umwandelt, z.B. indem man eine Verbindung der Formel I (R³ = H) mit einer Verbindung der Formel E-R³ (worin R³ von H verschieden ist) umsetzt oder Nitrogruppen (beispielsweise durch Hydrierung an Raney-Nickel oder Pd-Kohle in einem inerten Lösungsmittel wie Methanol oder Ethanol) zu Aminogruppen reduziert und/oder freie Amino- und/oder Hydroxygruppen funktionell abwandelt und/oder funktionell abgewandelte Amino- und/oder Hydroxygruppen durch Solvolyse oder Hydrogenolyse freisetzt und/oder Nitrilgruppen zu COOH-Gruppen hydrolysiert und/oder Thioethergruppen zu SO- oder SO₂-Gruppen oxydiert, z.B. mit H₂O₂ oder einer Persäure wie 3-Chlorperbenzoesäure und/oder Verbindungen der Formel I, welche eine Carbonylgruppe enthalten, in Verbindungen der Formel I umwandeln, welche eine -C(=NR¹⁵)-Gruppe enthalten, z.B. durch Umsetzung mit einer Verbindung der Formel H₂N-R¹⁵ wie Ammoniak, Hydroxylamin, O-Alkyl-, O-Alkenyl-, O-Alkinyl- oder O-Aryl-hydroxylaminen, Cyanamid oder primären Aminen der Formel H₂N-R¹⁶, eine Carbonsäuregruppe verestert oder amidiert, z.B. durch Umsetzung mit einem Alkohol der Formel A-OH oder mit einem Amin der Formel HNR⁹R¹⁰ oder der Formel H₂N-CHR¹⁸-COOA, und/oder eine Carbamidoximgruppe durch Umsetzung (a) mit 1,1'-Carbonyldiimidazol oder einem Chlorameisensäurealkylester in eine 4,5-Dihydro-5-oxo-1,2,4-oxadiazol-3-yl-gruppe, (b) mit 1,1'-Thiocarbonyl-diimidazol in eine 4,5-Dihydro-5-oxo-1,2,4-thiadiazol-3-yl-gruppe, (c) mit SOCl₂ in eine 2-Oxo-3H-1,2,3,5-oxathiadiazol-4-yl-gruppe oder (d) mit SO₂Cl₂ in eine 2,2-Dioxo-3H-1,2,3,5-oxathiadiazol-4-yl-gruppe umwandelt und/oder eine -SO₂NH-COOR⁵-Gruppe durch Amidierung mit einer Verbindung der Formel HNR⁵R⁶ in eine -SO₂NH-CO-NR⁵R⁶-Gruppe umwandelt.

So kann man Verbindungen der Formel I (R³ = H) durch Reaktion mit Verbindungen der Formel E-R³ alkylieren. Man arbeitet dabei bevorzugt in einem inerten Lösungsmittel, z.B. einem Säureamid wie DMF, N-Methylpyrrolidon, 1,3-Dimethyl-2-oxohexahydropyrimidin oder Phosphorsäure-hexamethyl-triamid, einem Alkohol wie Methanol oder tert.-Butanol, einem Ether wie THF oder einem halogenierten Kohlenwasserstoff wie Dichlormethan oder Gemischen davon als Lösungsmittel und/oder in Gegenwart eines Alkalimetallalkoholats wie Natriummethylat oder Kalium-tert.-butylat, eines Alkalimetallhydrids wie Natrium- oder Kaliumhydrid, eines Alkalimetallcarbonats wie Natrium- oder Kaliumcarbonat, eines Alkalimetallbicarbonats wie Natrium- oder Kaliumbicarbonat oder eines tertiären Amins wie Triethylamin oder Ethyldiisopropylamin bei Temperaturen zwischen etwa -30 und 200, vorzugsweise zwischen 20 und 60°.

Ferner kann man freie Aminogruppen in üblicher Weise mit einem Säurechlorid oder -anhydrid acylieren oder mit einem unsubstituierten oder substituierten Alkylhalogenid alkylieren, zweckmäßig in einem inerten Lösungsmittel wie Dichlormethan oder THF und/oder in Gegenwart einer Base wie Triethylamin oder Pyridin bei Temperaturen zwischen -60 und +30°.

Gewünschtenfalls kann in einer Verbindung der Formel I eine funktionell angewandelte Amino- und/oder Hydroxygruppe durch Solvolyse oder Hydrogenolyse nach üblichen Methoden in Freiheit gesetzt werden. So kann z.B. eine Verbindung der Formel I, die eine NHCOR⁵- oder eine COOA-Gruppe enthält, in die entsprechende Verbindung der Formel I umgewandelt werden, die stattdessen eine NH₂- oder eine HOOC-Gruppe enthält. COOA-Gruppen können z.B. mit NaOH oder KOH in Wasser, Wasser-THF oder Wasser-Dioxan bei Temperaturen zwischen 0 und 100° verseift werden.

Bei der Amidierung von Carbonsäuregruppen arbeitet man zweckmäßig nach üblichen Methoden der Peptid-Synthese, wie sie z.B. in Houben-Weyl, 1.c., Band 15/II, Seiten 1-806 (1974) beschrieben sind. Die Reaktion gelingt vorzugsweise in Gegenwart eines Dehydratisierungsmittels, z.B. eines Carbodiimids wie N,N'-Dicyclohexylcarbodiimid ("DCCI"), 1,1'-Carbonyl-diimidazol oder N-3-Dimethylaminopropyl-N'-ethyl-carbodiimid ("DAPECI"), ferner Propanphosphonsäureanhydrid (vgl. Angew.Chem. 92, 129 (1980)), Diphenylphosphorylazid oder 2-Ethoxy-N-ethoxycarbonyl-1,2-dihydrochinolin, in einem inerten Lösungsmittel, z.B. einem halogenierten Kohlenwasserstoff wie Dichlormethan, einem Ether wie THF oder Dioxan, einem Amid wie DMF oder Dimethylacetamid, einem Nitril wie Acetonitril, bei Temperaturen zwischen etwa -10 und 40, vorzugsweise zwischen 0 und 30°.

An Stelle der Carbonsäuren können auch geeignete reaktionsfähige Derivate dieser Stoffe in die Reaktion eingesetzt werden, z.B. solche, in denen reaktive Gruppen intermediär durch Schutzgruppen blockiert sind. Die Säuren können z.B. in Form ihrer aktivierten Ester verwendet werden, die zweckmäßig in situ gebildet werden, z.B. durch Zusatz von 1-Hydroxybenzotriazol oder N-Hydroxysuccinimid.

Die oben genannten Umwandlungen einer Carbamidoximgruppe (I, R² = -C(NH₂)=NOH) in verschiedene oben genannte heterocyclische Reste erfolgt bevorzugt in Gegenwart eines inerten Lösungsmittels, z.B. eines Ethers wie THF, eines Kohlenwasserstoffs wie Toluol, eines Amids wie DMF, eines halogenierten Kohlenwasserstoffs wie Dichlormethan oder einer Base wie Pyridin oder eines Gemisches zweier dieser Lösungsmittel bei Temperaturen zwischen 0 und 100°.

Eine Base der Formel I kann mit einer Säure in das zugehörige Säureadditionssalz übergeführt werden, beispielsweise durch Umsetzung äquivalenter Mengen der Base und der Säure in einem inerten Lösungsmittel wie Ethanol und anschließendes Eindampfen. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure; 2-Hydroxyethansulfonsäure, Benzolsulfonsäure, p-Toluolsulfonsäure, Naphthalin-mono- und disulfonsäuren, Laurylschwefelsäure. Salze mit physiologisch nicht unbedenklichen Säuren, z.B. Pikrate, können zur Isolierung und/oder Aufreinigung der Verbindung der Formel I verwendet werden.

Andererseits können Verbindungen der Formel I, die COOH- oder z.B. 1,2,4-Oxadiazolgruppen enthalten, mit Basen (z.B. Natrium- oder Kaliumhydroxid oder -carbonat) in die entsprechenden Metall-, insbesondere Alkalimetall- oder Erdalkalimetall-, oder in die entsprechenden Ammoniumsalze umgewandelt werden. Die Kaliumsalze der 1,2,4-Oxadiazolderivate sind besonders bevorzugt.

Die neuen Verbindungen der Formel I und ihre physiologisch unbedenklichen Salze können zur Herstellung pharmazeutischer Präparate verwendet werden, indem man sie zusammen mit mindestens einem Träger- oder Hilfsstoff und, falls erwünscht, zusammen mit einem oder mehreren weiteren Wirkstoff(en) in eine geeignete Dosierungsform bringt. Die so erhaltenen Zubereitungen können als Arzneimittel in der Human- oder Veterinärmedizin eingesetzt werden. Als Trägersubstanzen kommen organische oder anorganische Stoffe in Frage, die sich für die enterale (z.B. orale oder rektale) oder parenterale Applikation oder für eine Applikation in Form eines Inhalations-Sprays eignen und mit den neuen Verbindungen nicht reagieren, beispielsweise Wasser, pflanzliche Öle, Benzylalkohole, Polyethylenglykole, Glycerintriacetat und andere Fettsäureglyceride, Gelatine, Sojalecithin, Kohlehydrate wie Lactose oder Stärke, Magnesiumstearat, Talk, Cellulose. Zur oralen Anwendung dienen insbesondere Tabletten, Dragees, Kapseln, Sirupe, Säfte oder Tropfen; von Interesse sind spezielle Lacktabletten und Kapseln mit magensaftresistenten Überzügen bzw. Kapselhüllen. Zur rektalen Anwendung dienen-Suppositorien, zur parenteralen Applikation Lösungen, vorzugsweise ölige oder wässerige Lösungen, ferner Suspensionen, Emulsionen oder Implantate. Für die Applikation als Inhalations-Spray können Sprays verwendet werden, die den Wirkstoff entweder gelöst oder suspendiert in einem Treibgasgemisch enthalten. Zweckmäßig verwendet man den Wirkstoff dabei in mikronisierter Form, wobei ein oder mehrere zusätzliche physiologisch verträgliche Lösungsmittel zugegen sein können, z.B. Ethanol. Inhalationslösungen können mit Hilfe üblicher Inhalatoren verabfolgt werden. Die neuen Verbindungen können lyophilisiert und die erhaltenen Lyophilisate z.B. zur Herstellung von Injektionspräparaten verwendet werden. Die angegebenen Zubereitungen können sterilisiert sein und/oder Hilfsstoffe wie Konservierungs-, Stabilisierungs- und/oder Netzmittel, Emulgatoren, Salze zur Beinflussung des osmotischen Druckes, Puffersubstanzen, Farb- und/oder Aromastoffe enthalten. Sie können, falls erwünscht, auch einen oder mehrere weitere Wirkstoffe enthalten, z.B. ein oder mehrere Vitamine, Diuretika, Antiphlogistika.

Die erfindungsgemäßen Substanzen werden in der Regel in Analogie zu anderen bekannten, im Handel befindlichen Präparaten, z.B. Captopril oder Enalapril, insbesondere aber in Analogie zu den in der US-PS 4 880 804 beschriebenen Verbindungen verabreicht, vorzugsweise in Dosierungen zwischen etwa 1 mg und 1 g, insbesondere zwichen 50 und 500 mg pro Dosierungseinheit. Die tägliche Dosierung liegt vorzugsweise zwischen etwa 0,1 und 50 mg/kg, insbesondere 1 und 10 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Vor und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und/oder durch Kristallisation.
IP = Imidazo[4,5-c]pyridin. Rf-Werte an Kieselgel; Laufmittel: Dichlormethan/Methanol 95:5.

### Beispiel 1

Ein Gemisch von 0,7 g K₂CO₃, 3,04 g 2-Butyl-5-(N,N-diethylcarbamoylmethyl)-4,5-dihydro-4-oxo-3H-IP (erhältlich durch Kondensation von Valeriansäure mit 3,4-Diamino-2-chlorpyridin in Gegenwart von Polyphosphorsäure zu 2-Butyl-4,5-dihydro-4-oxo-1(oder3)H-IP, Reaktion mit Benzylbromid in Methanol in Gegenwart von CH₃ONa zu 3-Benzyl-2-butyl-4,5-dihydro-4-oxo-3H-IP, Reaktion mit N,N-Diethyl-chloracetemid in DMF in Gegenwart von K-tert.-butylat zu 3-Benzyl-2-butyl-5-(N,N-diethylcarbamoyl-methyl)-4,5-dihydro-4-oxo-3H-IP und hydrogenolytische Abspaltung der Benzylgruppe) und 30 ml DMF wird 1 Std. bei 20° gerührt. Anschließend tropft man bei 0° unter Rühren eine Lösung von 3,31 g 4-Brommethyl-2'-(4,5-dihydro-5-oxo-1,2,4-oxadiazol-3-yl)-biphenyl (erhältlich durch Reaktion von 4-Methyl-2'-cyan-biphenyl mit Hydroxylamin zu 4-Methyl-2'-(amino-oximinomethyl)-biphenyl, Umsetzung mit Ethyl-chlorformiat zu 4-Methyl-2'-(4,5-dihydro-5-oxo-1,2,4-oxadiazol-3-yl)-biphenyl und Bromierung) in 35 ml DMF hinzu. Man rührt 16 Std. bei 20°, dampft ein, arbeitet wie üblich auf und erhält 2-Butyl-3-(2'-(4,5-dihydro-5-oxo-1,2,4-oxadiazol-3-yl)-biphenylyl-4-methyl)-4,5-dihydro-4-oxo-5-(N,N-diethylcarbamoylmethyl)-3H-IP, F. 142°.
K-Salz, F. 275° (Zers.).

### Beispiel 2

Ein Gemisch von 1,02 g Valeriansäure, 5 g 4-Amino-1,2-dihydro-2-oxo-3-(2'-(4,5-dihydro-5-oxo-1,2,4-oxadiazol-3-yl)-biphenylyl-4-methyl-amino)-1-piperidinocarbonylmethyl)-pyridin (erhältlich durch Reaktion von 3-Amino-4-benzylamino-1,2-dihydro-2-oxo-1-piperidinocarbonylmethyl-pyridin mit 4-Brommethyl-2'-(4,5-dihydro-5-oxo-1,2,4-oxadiazol-3-yl)-biphenyl und hydrogenolytische Abspaltung der Benzylgruppe) und 50 g Polyphosphorsäure wird 5 Std. auf 140° erhitzt. Als Zwischenprodukte entstehen in situ 4-Amino-1,2-dihydro-2-oxo-3-(N-(2'-(4,5-dihydro-5-oxo-1,2,4-oxadiazol-3-yl)-biphenyl-4-methyl)-N-valeryl-amino)-1-piperidinocarbonylmethyl-pyridin und 1,2-Dihydro-2-oxo-3-(2'-(4,5-dihydro-5-oxo-1,2,4-oxadiazol-3-yl)-biphenylyl-4-methyl-amino)-1-piperidinocarbonylmethyl-4-valerylamino-pyridin Man kühlt ab, gießt auf Eis, macht mit Natronlauge alkalisch, arbeitet wie üblich auf und erhalt 2-Butyl-3-(2'-(4,5-dihydro-5-oxo-1,2,4-oxadiazol-3-yl)-biphenylyl-4-methyl)-4,5-dihydro-4-oxo-5-piperidino-carbonylmethyl-3H-IP. K-Salz, F. 284° (Zers.)

Analog erhält man mit
Essigsäure
Propionsäure
Buttersäure
Cyclopropylcarbonsäure
Cyclopropylessigsäure
an Stelle von Valeriansäure die nachstehenden 3-(2'-(4,5-Dihydro-5-oxo-1,2,4-oxadiazol-3-yl)-biphenylyl-4-methyl)-4,5-dihydro-4-oxo-5-piperidinocarbonylmethyl-3H-IP:
2-Methyl-
2-Ethyl-
2-Propyl-
2-Cyclopropyl-
2-Cyclopropylmethyl-.

### Beispiel 3

Ein Gemisch von 3,86 g 2-Butyl-3-p-aminobenzyl-4,5-dihydro-4-oxo-5-benzyl-3H-IP (erhältlich durch Reaktion von 2-Butyl-4,5-dihydro-4-oxo-5-benzyl-3H-IP-mit p-Nitrobenzylbromid zu 2-Butyl-3-p-nitrobenzyl-4,5-dihydro-4-oxo-5-benzyl-3H-IP und anschließende Reduktion der Nitrogruppe mit HCl/Sn), 3 ml Triethylamin, 0,5 g 4-Dimethylaminopyridin und 120 ml Dichlormethan wird auf 5° gekühlt und tropfenweise mit einer Lösung von 2,25 g o-(4,5-Dihydro-5-oxo-1,2,4-oxadiazol-3-yl)-benzoylchlorid in 20 ml Dichlormethan versetzt. Man rührt noch 16 Std. bei 20°, dampft ein, arbeitet wie üblich auf und erhält 2-Butyl-3-p-(o-(4,5-dihydro-5-oxo-1,2,4-oxadiazol-3-yl)-benzamido)-benzyl-4,5-dihydro-4-oxo-5-benzyl-3H-IP.

### Beispiel 4

Ein Gemisch von 4,15 g 2-Butyl-3-p-carboxybenzyl-4,5-dihydro-4-oxo-5-benzyl-3H-IP, 12 g Thionylchlorid und 35 ml CHCl₃ wird 6 Std. gekocht und dann eingedampft. Das erhaltene rohe Säurechlorid wird durch mehrfaches Lösen in Toluol und Eindampfen von Thionylchloridresten befreit und in 80 ml THF gelöst. Man tropft diese Lösung zu einer Lösung von 1,77 g 3-o-Aminophenyl-4,5-dihydro-1,2,4-oxadiazol-5-on und 0,4 g NaOH in 100 ml Wasser, rührt 24 Std. und arbeitet wie üblich auf. Man erhält 2-Butyl-3-(p-(o-(4,5-dihydro-5-oxo-1,2,4-oxadiazol-3-yl)-anilinocarbonyl)-benzyl)-4,5-dihydro-4-oxo-5-benzyl-3H-IP.

### Beispiel 5

a) Eine Lösung von 7,1 g Hydroxylammoniumchlorid in 30 ml DMSO wird mit 14,4 ml Triethylamin in 40 ml THF versetzt. Man filtriert, dampft das Filtrat ein und versetzt es mit 3,82 g 2-Butyl-3-(2'-cyan-biphenylyl-4-methyl)-4,5-dihydro-4-oxo-3H-IP sowie weiteren 3,5 ml Triethylamin. Die Lösung wird 16 Std. bei 75° gerührt. Man kühlt ab, gießt in Wasser, filtriert das ausgefallene 2-Butyl-3-(2-(amino-oximino-methyl)-biphenylyl-4-methyl)-4,5-dihydro-4-oxo-3H-IP ab und reinigt durch Chromatographie. Rf 0,28 (Kieselgel; Methyl-tert.-butyl-ether/Methanol 9:1).
b) Eine Suspension von 4,15 g dieser Verbindung in 32 ml THF wird mit 2,4 g 1,1'-Carbonyldiimidazol versetzt und 7 Std. unter Rühren gekocht. Man dampft ein, arbeitet wie üblich auf (Ethylacetat/verdünnte H₂SO₄) und erhält 2-Butyl-3-(2'-(4,5-dihydro-5-oxo-1,2,4-oxadiazol-3-yl)-biphenylyl-4-methyl)-4,5-dihydro-4-oxo-3H-IP. F. 244°. K-Salz, F. 186-190°.

Analog a) erhält man aus 2-Butyl-3-(2'-cyan-biphenylyl-4-methyl)-4,5-dihydro-4-oxo-5-(N,N-diethylcarbamoylmethyl)-3H-IP das 2-Butyl-3-(2'-(amino-oximino-methyl)-biphenylyl-4-methyl)-4,5-dihydro-4-oxo-5-(N,N-diethylcarbamoyl-methyl)-3H-IP, F. 220°, und daraus analog b) das 2-Butyl-3-(2'-(4,5-dihydro-5-oxo-1,2,4-oxadiazol-3-yl)-biphenylyl-4-methyl)-4,5-dihydro-4-oxo-5-(N,N-diethylcarbamoyl-methyl)-3H-IP, F. 142°.

### Beispiel 6

a) Zu einer Lösung von 526 mg 2-Butyl-3-(2'-aminosulfonyl-biphenylyl-4-methyl)-4,5-dihydro-4-oxo-5-benzyl-3H-IP ["A"; F.153°; erhältlich durch Reaktion von 2-Butyl-4-oxo-4,5-dihydro-1(oder 3)H-IP mit 4'-Brommethyl-biphenyl-2-sulfonsäure-(N-tert.-butylamid) zu 2-Butyl-3-(2'-N-tert.-butyl-aminosulfonyl-biphenylyl-4-methyl)-4,5-dihydro-4-oxo-3H-IP ("B"; FAB 493), Umsetzung mit Benzylbromid/K-tert.-butanolat in DMF bei 20° zu 2-Butyl-3-(2'-N-tert.-butyl-aminosulfonyl-biphenylyl-4-methyl)-4,5-dihydro-4-oxo-5-benzyl-3H-IP (F. 71°) und Abspaltung der tert.-Butylgruppe mit CF₃COOH/Anisol] und 360 mg 4-Dimethylaminopyridin in 12 ml Pyridin gibt man 550 mg Ethylchlorformiat, rührt das Gemisch 48 Std. bei 20°, gibt 8 ml Methanol hinzu und arbeitet wie üblich auf. Man erhält 2-Butyl-3-(2'-(N-ethoxycarbonyl-aminosulfonyl)-biphenylyl-4-methyl)-4,5-dihydro-4-oxo-5-benzyl-3H-IP. F. 95°.

Analog erhält man aus "A" und Butylchlorformiat das 2-Butyl-3-(2'-(N-butoxycarbonyl-aminosulfonyl)-biphenylyl-4-methyl)-4,5-dihydro-4-oxo-5-benzyl-3H-IP, F. 79°.

Analog erhält man aus "B" mit den entsprechenden Halogeniden (z.B. Ethylbromid) die nachstehenden 2-Butyl-3-(2'-(N-tert.-butyl-aminosulfonyl)-biphenylyl-4-methyl)-4,5-dihydro-4-oxo-5-R³-3H-IP:
-5-ethyl-
-5-carboxymethyl-
-5-methoxycarbonylmethyl-
-5-ethoxycarbonylmethyl-
-5-carbamoylmethyl-
-5-N,N-dimethyl-carbamoylmethyl-
-5-N,N-diethyl-carbamoylmethyl-
-5-N,N-diphenyl-carbamoylmethyl-
-5-N-phenyl-carbamoylmethyl-
-5-N-methyl-N-phenyl-carbamoylethyl-
-5-(2-oxo-3,3-dimethylbutyl)-
-5-phenacyl-
-5-(2-carboxy-phenacyl)-
-5-(2-methoxy-phenacyl)-
-5-pyrrolidinocarbonylmethyl-
-5-piperidinocarbonylmethyl-
-5-morpholinocarbonylmethyl-
-5-(2-methoxycarbonyl-benzyl)-
-5-(2-ethoxycarbonyl-benzyl)-
-5-(2-chlorbenzyl)-
-5-(2-chlor-6-nitro-benzyl)-
-5-(2-thienylmethyl)-
-5-cinnamyl-
-5-(α-methoxycarbonyl-benzyl)-
-5-(α-isopropoxycarbonyl-benzyl)-
-5-phenylthiomethyl-
-5-phenylsulfonylmethyl-,
daraus die nachstehenden 2-Butyl-3-(2'-(aminosulfonyl)-biphenylyl-4-methyl)-4,5-dihydro-4-oxo-5-R³-3H-IP:
-5-ethyl-
-5-carboxymethyl-
-5-methoxycarbonylmethyl-
-5-ethoxycarbonylmethyl-
-5-carbamoylmethyl-
-5-N,N-dimethyl-carbamoylmethyl-
-5-N,N-diethyl-carbamoylmethyl-
-5-N,N-diphenyl-carbamoylmethyl-
-5-N-phenyl-carbamoylmethyl-
-5-N-methyl-N-phenyl-carbamoylmethyl-
-5-(2-oxo-3,3-dimethylbutyl)-
-5-phenacyl-
-5-(2-carboxy-phenacyl)-
-5-(2-methoxy-phenacyl)-
-5-pyrrolidinocarbonylmethyl-
-5-piperidinocarbonylmethyl-
-5-morpholinocarbonylmethyl-
-5-(2-methoxycarbonyl-benzyl)-
-5-(2-ethoxycarbonyl-benzyl)-
-5-(2-chlorbenzyl)-
-5-(2-chlor-6-nitro-benzyl)-
-5-(2-thienylmethyl)-
-5-cinnamyl-
-5-(α-methoxycarbonyl-benzyl)-
-5-(α-isopropoxycarbonyl-benzyl)-
-5-phenylthiomethyl-
-5-phenylsulfonylmethyl-,
und daraus die nachstehenden 2-Butyl-3-(2'-(N-butoxycarbonylaminosulfonyl)-biphenylyl-4-methyl)-4,5-dihydro-4-oxo-5-R³-3H-IP:
-5-ethyl-
-5-carboxymethyl-
-5-methoxycarbonylmethyl-
-5-ethoxycarbonylmethyl-
-5-carbamoylmethyl-
-5-N,N-dimethyl-carbamoylmethyl-, F. 146°
-5-N,N-diethyl-carbamoylmethyl-
-5-N,N-diphenyl-carbamoylmethyl-, F. 92°
-5-N-phenyl-carbamoylmethyl-
-5-N-methyl-N-phenyl-carbamoylmethyl-
-5-(2-oxo-3,3-dimethylbutyl)-
-5-phenacyl-
-5-(2-carboxy-phenacyl)-
-5-(2-methoxy-phenacyl)-
-5-pyrrolidinocarbonylmethyl-
-5-piperidinocarbonylmethyl-
-5-morpholinocarbonylmethyl-
-5-(2-methoxycarbonyl-benzyl)-, F. 77°
-5-(2-ethoxycarbonyl-benzyl)-
-5-(2-chlorbenzyl)-
-5-(2-chlor-6-nitro-benzyl)-
-5-(2-thienylmethyl)-
-5-cinnamyl-
-5-(α-methoxycarbonyl-benzyl)-
-5-(α-isopropoxycarbonyl-benzyl)-, F. 80°
-5-phenylthiomethyl-
-5-phenylsulfonylmethyl-.

Analog erhält man mit Acetylchlorid, Propionylchlorid, Butyrylchlorid, Cyclopropylcarbonylchlorid, Cyclopropylacetylchlorid, Cyclopentylcarbonylchlorid, Benzoylchlorid, p-Methoxybenzoylchlorid, p-Chlorbenzoylchlorid oder 2-Thienylcarbonylchlorid an Stelle von Ethylchlorformiat die nachstehenden 2-Butyl-3-(2'-R²-biphenylyl-4-methyl)-4,5-dihydro-4-oxo-5-benzyl-3H-IP:
-3-(2'-(acetyl-aminosulfonyl)-biphenylyl-4-methyl)-
-3-(2'-(propionyl-aminosulfonyl)-biphenylyl-4-methyl)-
-3-(2'-(butyryl-aminosulfonyl)-biphenylyl-4-methyl)-
-3-(2'-(cyclopropylcarbonyl-aminosulfonyl)-biphenylyl-4-methyl)-, F. 142°
-3-(2'-(cyclopropylacetyl-aminosulfonyl)-biphenylyl-4-methyl)-
-3-(2'-(cyclopentylcarbonyl-aminosulfonyl)-biphenylyl-4-methyl)-
-3-(2'-(benzoyl-aminosulfonyl)-biphenylyl-4-methyl)-
-3-(2'-(p-methoxybenzoyl-aminosulfonyl)-biphenylyl-4-methyl)-
-3-(2'-(p-chlorbenzoyl-aminosulfonyl)-biphenylyl-4-methyl)-
-3-(2'-(2-thienylcarbonyl-aminosulfonyl)-biphenylyl-4-methyl)-.

Analog erhält man aus den entsprechenden Ausgangsstoffen die nachstehenden 2-Butyl-3-(2'-R²-biphenylyl-4-methyl)-4,5-dihydro-4-oxo-5-R³-3H-IP:
-3-(2'-(cyclopropylcarbonyl-aminosulfonyl)-biphenylyl-4-methyl)-5-(2-ethoxycarbonyl-benzyl)-, F. 118°
-3-(2'-(benzoyl-aminosulfonyl)-biphenylyl-4-methyl)-5-N,N-dimethyl-carbamoylmethyl-, F. 209°
-3-(2'-(trifluoracetyl-aminosulfonyl)-biphenylyl-4-methyl)-5-(2-chlorbenzyl)-, F. 121°
b) Eine Lösung von 598 mg der nach (a) erhaltenen Ethoxycarbonylverbindung und 108 mg 2-Aminomethylpyridin in 30 ml Toluol wird 2 Std. gekocht, abgekühlt und wie üblich aufgearbeitet. Man erhält 2-Butyl-3-(2'-N-(N-2-pyridyl-methyl-carbamoyl)-aminosulfonyl-biphenylyl-4-methyl)-4,5-dihydro-4-oxo-5-benzyl-3H-IP.

### Beispiel 7

Ein Gemisch von 526 mg "A" und 7 ml Phenylisocyanat wird 4 Std. auf 80° erhitzt. Man kühlt ab, destilliert das überschüssige Phenylisocyanat ab und erhält nach chromatographischer Aufreinigung 2-Butyl-3-(2'-N-(N-phenylcarbamoyl)aminosulfonyl-biphenylyl-4-methyl)-4,5-dihydro-4-oxo-5-benzyl-3H-IP.

### Beispiel 8

Eine Lösung von 4,41 g 2-Butyl-3-(2'-(4,5-dihydro-5-oxo-1,2,4-oxadiazol-3-yl)-biphenylyl-4-methyl)-4,5-dihydro-4-oxo-3H-IP (siehe Beispiel 5) in 35 ml DMF wird unter Rühren bei 20° mit 1,25 g K-tert.-butylat versetzt. Nach 45 Min. Rühren wird eine Lösung von 1,27 g Benzylchlorid in 15 ml DMF zugetropft. Man rührt noch 16 Std. bei 20°, arbeitet wie üblich auf und erhält 2-Butyl-3-(2'-(4,5-dihydro-5-oxo-1,2,4-oxadiazol-3-yl)-biphenylyl-4-methyl)-4,5-dihydro-4-oxo-5-benzyl-3H-IP, F. 220° ("C").

Analog erhält man die nachstehenden 2-Butyl-3-(2'-(4,5-dihydro-5-oxo-1,2,4-oxadiazol-3-yl)-biphenylyl-4-methyl)-4,5-dihydro-4-oxo-5-R³-3H-IP:
mit Ethyliodid:
-5-ethyl-
mit Bromessigsäure:
-5-carboxymethyl- ("E"), F. 203°
mit Bromessigsäuremethylester:
-5-methoxycarbonylmethyl-
mit Bromessigsäureethylester:
-5-ethoxycarbonylmethyl-
mit Bromessigsäure-tert.butylester:
-5-tert.butoxycarbonylmethyl-, F. 168°; K-Salz, F.219°
mit Bromacetamid:
-5-carbamoylmethyl-, F. 156°; K-Salz, F. 195°
mit N,N-Dimethyl-chloracetamid:
-5-N,N-dimethyl-carbamoylmethyl-, Rf. 0,25; K-Salz, F. 189°
mit N,N-Diethyl-chloracetamid:
-5-N,N-diethyl-carbamoylmethyl-, F 142°
mit N,N-Diphenyl-chloracetamid:
-5-N,N-diphenyl-carbamoylmethyl-
mit N-Phenyl-chloracetamid:
-5-N-phenyl-carbamoylmethyl-
mit N-Methyl-N-phenylchloracetamid:
-5-N-methyl-N-phenyl-carbamoylmethyl-
mit 2-Oxo-3,3-dimethyl-butylbromid:
-5-(2-oxo-3,3-dimethyl-butyl)-
mit Phenacylbromid:
-5-phenacyl-
mit 2-Carboxy-phenacylbromid:
-5-(2-carboxy-phenacyl)-
mit 2-Methoxy-phenacylbromid:
-5-(2-methoxy-phenacyl)-
mit Bromessigsäurepyrrolidid:
-5-pyrrolidinocarbonylmethyl-
mit Bromessigsäurepiperidid:
-5-piperidinocarbonylmethyl-, K-Salz, F. 284° (Zers.)
mit Bromessigsäuremorpholid:
-5-morpholinocarbonylmethyl-
mit 2-Brommethyl-benzoesäureethylester:
-5-(2-ethoxycarbonyl-benzyl)-
mit 2-Chlor-benzylbromid:
-5-(2-chlorbenzyl)-
mit 2-Chlor-6-nitro-benzylbromid:
-5-(2-chlor-6-nitro-benzyl)-
mit 2-Thienylmethylchlorid:
-5-(2-thienylmethyl)-
mit Cinnamylbromid:
-5-cinnamyl-
mit α-Brom-phenylessigsäuremethylester:
-5-(α-methoxycarbonyl-benzyl)-
mit α-Brom-phenylessigsäureisopropylester
-5-(α-isopropoxycarbonyl-benzyl)-, F. 175°
mit α-Brom-phenylessigsäure-piperidid:
-5-(α-piperidinocarbonyl-benzyl)-, Rf. 0,45; K-Salz, F. 193°
mit Phenylthiomethylchlorid:
-5-phenylthiomethyl-
mit Phenylsulfonylmethylchlorid:
-5-phenylsulfonylmethyl-
mit 1-Brom-3-nitro-aceton:
-5-(3-nitro-2-oxo-propyl)-
mit 6-BOC-amino-1-chlor-2-hexanon:
-5-(6-BOC-amino-2-oxo-hexyl)-.

### Beispiel 9

Eine Suspension von 0,7 g 2-Butyl-3-(2'-amino-oximino-methyl-biphenylyl-4-methyl)-4,5-dihydro-4-oxo-5-benzyl-3H-IP ["D"; F. 224°; erhältlich durch Reaktion von 2-Butyl-3-(2'-cyan-biphenylyl-4-methyl)-4-oxo-5-benzyl-4,5-dihydro-3H-imidazo[4,5-c]pyridin mit Hydroxylamin in Ethanol/Wasser] in 20 ml Toluol wird mit 0,13 ml Ethylchlorformiat versetzt und 18 Std. bei 60° gerührt. Man setzt dann 10 ml DMF hinzu, rührt weitere 18 Std., gibt anschließend 1,3 ml 1N NaOH hinzu, kocht 4 Std., kühlt ab, stellt auf pH 4, arbeitet wie üblich auf und erhält "C", F. 220°.

### Beispiel 10

Man suspendiert 5,05 g "D" unter Argon in 70 ml Pyridin und versetzt bei 0° unter Rühren tropfenweise mit einer Lösung von 0,85 ml SOCl₂ in 35 ml CH₂Cl₂. Man rührt noch 2 Std. bei 0°, gießt in 500 ml Wasser, arbeitet wie üblich auf und erhält 2-Butyl-3-(2'-(2-oxo-3H-1,2,3,5-oxathiadiazol-4-yl)-biphenylyl-4-methyl)-4,5-dihydro-4-oxo-5-benzyl-3H-IP.

### Beispiel 11

Analog Beispiel 10 erhält man aus "D" und SO₂Cl₂ das 2-Butyl-3-(2'-(2,2-dioxo-3H-1,2,3,5-oxathiadiazol-4-yl)-biphenylyl-4-methyl)-4,5-dihydro-4-oxo-5-benzyl-3H-IP.

### Beispiel 12

Ein Gemisch von 505 mg "D", 10 ml THF und 180 mg 1,1'-Thiocarbonyldiimidazol wird 30 Min. bei 20° gerührt und eingedampft. Man löst den Rückstand in Ethylacetat, wäscht mit verdünnter Salzsäure, dann mit Wasser, trocknet und dampft erneut ein. Der so erhaltene Rückstand wird in einem Gemisch von 60 ml Chloroform und 12 ml Methanol gelöst. Zu dieser Lösung gibt man 3,5 g Kieselgel, rührt 48 Std. bei 20°, filtriert, dampft ein und reinigt chromatographisch an Kieselgel. Man erhält 2-Butyl-3-(2'-(4,5-dihydro-5-oxo-1,2,4-thiadiazol-3-yl)-biphenylyl-4-methyl)-4,5-dihydro-4-oxo-5-benzyl-3H-IP.

### Beispiel 13

Man löst 5,05 g "D" in 50 ml Dichlormethan, gibt 1,15 g Triethylamin und 1,15 g Acetanhydrid hinzu und rührt 2 Std. bei 20°. Man dampft ein, arbeitet wie üblich auf (Ethylacetat/Wasser; Waschen mit NaHCO₃-Lösung) und löst das erhaltene rohe Acetylderivat in 30 ml DMF. Man gibt zunächst 4 g CS₂, dann innerhalb von 10 Minuten NaH (60 %, in Öl; 1,4 g) hinzu und rührt 2 Std. bei 20°. Nach üblicher Aufarbeitung (pH 3, Eiswasser) erhält man 2-Butyl-3-(2'-(4,5-dihydro-5-thioxo-1,2,4-oxadiazol-3-yl)-biphenylyl-4-methyl)-4,5-dihydro-4-oxo-5-benzyl-3H-IP.

### Beispiel 14

Man tropft unter Eiskühlung 1,5 ml 1N NaOH zur einer Lösung von 1 g 2-Butyl-3-(2'-(5-trichlomethyl-1,2,4-oxadiazol-3-yl)-biphenylyl-4-methyl)-4,5-dihydro-4-oxo-5-benzyl-3H-IP (erhältlich aus 3-(4'-Brommethyl-2-biphenylyl)-5-trichlormethyl-1,2,4-oxadiazol (EP-A2-520423, Beispiel 22 c) und 2-Butyl-4,5-dihydro-4-oxo-5-benzyl-3H-IP analog Beispiel 1) in einem Gemisch von 8 ml Dioxan und 2 ml Wasser, rührt 30 Min. unter Eiskühlung, arbeitet wie üblich auf (verdünnte Salzsäure/Ethylacetat) und erhält "C", F. 220°.

### Beispiel 15

Zu einer Lösung von 499 mg "E" in 14 ml THF gibt man 210 mg DCCI, rührt 10 Min. bei 20°, setzt 72 mg Pyrrolidin zu und rührt weitere 18 Std. bei 20°. Man filtriert, arbeitet das Filtrat wie üblich auf, chromatographiert das Rohprodukt an Kieselgel (Ethylacetat/Methanol 80:20) und erhält 2-Butyl-3-(2'-(4,5-dihydro-5-oxo-1,2,4-oxadiazol-3-yl)-biphenylyl-4-methyl)-4,5-dihydro-4-oxo-5-pyrrolidinocarbonylmethyl-3H-IP.

### Beispiel 16

Zu einer Lösung von 5 g "E" und 2,44 g 1-p-Fluorphenylsulfonylpiperazin in 90 ml DMF gibt man nacheinander 1,94 g DAPECI, 1,36 g 1-Hydroxy-benzotriazol und 1,1 ml N-Methylmorpholin, rührt das Gemisch 5 Std. bei 20°, fällt mit Wasser das Produkt aus, filtriert ab, wäscht mit Wasser, trocknet und erhält 2-Butyl-3-(2'-(4,5-dihydro-5-oxo-1,2,4-oxadiazol-3-yl)-biphenylyl-4-methyl)-4,5-dihydro-4-oxo-5-(4-p-fluorphenylsulfonyl-piperazino-carbonylmethyl)-3H-IP.

### Beispiel 17

Eine Lösung von 5 g "E" in 20 ml THF wird unter Rühren zu einer Lösung von 1,6 g 1,1'-Carbonyldiimidazol in 20 ml THF zugetropft und anschließend 30 Min. erhitzt. Nach dem Abkühlen gibt man 1,6 g Benzolsulfonamid hinzu, rührt 10 Min., tropft eine Lösung von 1,48 g 1,8-Diazabicyclo[5,4,0]undec-7-en in 10 ml THF hinzu, rührt 18 Std. bei 20°, arbeitet wie üblich auf (1n Salzsäure/Dichlormethan) und erhält 2-Butyl-3-(2'-(4,5-dihydro-5-oxo-1,2,4-oxadiazol-3-yl)-biphenylyl-4-methyl)-4,5-dihydro-4-oxo-5-(N-phenylsulfonyl-carbamoylmethyl)-3H-IP.

### Beispiel 18

Ein Gemisch von 1 g 2-Butyl-3-(2'-(4,5-dihydro-5-oxo-1,2,4-oxadiazol-3-yl)-biphenylyl-4-methyl)-4,5-dihydro-4-oxo-5-ethoxycarbonylmethyl-3H-IP ("E"-Ethylester), 12 ml wässeriger 2 n NaOH-Lösung und 48 ml Methanol wird 2 Std. gekocht, dann eingedampft. Man arbeitet mit wässeriger Salzsäure/Dichlormethan wie üblich auf und erhält "E", F. 203°.

### Beispiel 19

Analog Beispiel 15 erhält man aus 2-Butyl-3-(2'-(4,5-dihydro-5-oxo-1,2,4-oxadiazol-3-yl)-biphenylyl-4-methyl)-4,5-dihydro-4-oxo-3H-IP-5-essigsäure und Diethylamin in Gegenwart von DCCI das 2-Butyl-3-(2'-(4,5-dihydro-5-oxo-1,2,4-oxadiazol-3-yl)-biphenylyl-4-methyl)-4,5-dihydro-4-oxo-5-(N,N-diethyl-carbamoylmethyl)-3H-IP, F. 142°.

### Beispiel 20

Eine Lösung von 1 g 2-Butyl-3-(2'-(4,5-dihydro-5-oxo-1,2,4-oxadiazol-3-yl)biphenylyl-4-methyl)-4,5-dihydro-4-oxo-5-(3-nitro-2-oxo-propyl)-3H-IP in 20 ml Methanol wird an 0,3 g 5%iger Pd-Kohle bei 20° und Normaldruck bis zur Aufnahme der berechneten H₂-Menge hydriert. Man filtriert den Katalysator ab, dampft ein und erhält 2-Butyl-3-[2'-(2'-(4,5-dihydro-5-oxo-1,2,4-oxadiazol-3-yl)-biphenylyl-4-methyl]-4,5-dihydro-4-oxo-5-(3-amino-2-oxo-propyl)-3H-IP.

### Beispiel 21

Eine Lösung von 1 g 2-Butyl-3-(2'-(4,5-dihydro-5-oxo-1,2,4-oxadiazol-3-yl)-biphenylyl-4-methyl)-4,5-dihydro-4-oxo-5-(6-BOC-amino-2-oxo-hexyl)-3H-IP in 20 ml Dichlormethan und 20 ml Trifluoressigsäure wird 1 Std. bei 20° gerührt, eingedampft und wie üblich aufgearbeitet. Man erhält 2-Butyl-3-(2'-(4,5-dihydro-5-oxo-1,2,4-oxadiazol-3-yl)-biphenylyl-4-methyl)-4,5-dihydro-4-oxo-5-(6-amino-2-oxo-hexyl)-3H-IP.

Die nachstehenden Beispiele betreffen pharmazeutische Zubereitungen, die Wirkstoffe der Formel I oder ihre Salze enthalten.

### Beispiel A: Tabletten und Dragees

In üblicher Weise werden Tabletten folgender Zusammensetzung gepreßt, die bei Bedarf mit einer üblichen Drageedecke auf Sucrosegrundlage überzogen werden:

| | |
|---|---|
| Wirkstoff der Formel I | 100 mg |
| Mikrokristalline Cellulose | 278,8 mg |
| Lactose | 110 mg |
| Maisstärke | 11 mg |
| Magnesiumstearat | 5 mg |
| Feinteiliges Siliciumdioxid | 0,2 mg |

### Beispiel B: Hartgelatine-Kapseln

Übliche zweiteilige Hartgelatine-Kapseln werden jeweils gefüllt mit

| | |
|---|---|
| Wirkstoff der Formel I | 100 mg |
| Lactose | 150 mg |
| Cellulose | 50 mg |
| Magnesiumstearat | 6 mg |

### Beispiel C: Weichgelatine-Kapseln

Übliche Weichgelatine-Kapseln werden mit einem Gemisch aus jeweils 50 mg Wirkstoff und 250 mg Olivenöl gefüllt.

### Beispiel D: Ampullen

Eine Lösung von 200 g Wirkstoff in 2 kg 1,2-Propandiol wird mit Wasser auf 10 l aufgefüllt und in Ampullen gefüllt, so daß jede Ampulle 20 mg Wirkstoff enthält.

### Beispiel E: Wässerige Suspension für orale Applikation

Eine wässerige Suspension des Wirkstoffs wird in üblicher Weise hergestellt. Die Einheitsdosis (5 ml) enthält 100 mg Wirkstoff, 100 mg Na-Carboxymethylcellulose, 5 mg Na-Benzoat und 100 mg Sorbit.

## Patentansprüche

1. Imidazopyridinderivate der Formel I 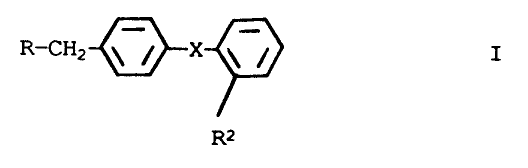 worin
R 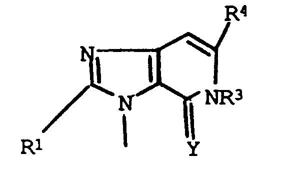
R¹ A, C₂₋C₆-Alkenyl, C₂-C₆-Alkinyl, C₃-C₈-Cycloalkyl-CₖH₂ₖ- oder C₁-C₆-Alkyl, worin eine CH₂-Gruppe durch O oder S ersetzt ist,
R² -SO₂NH-COOR⁵, -SO₂NH-COR⁵, -SO₂NH-SO₂R⁵, -SO₂NH-CONR⁵R⁶, -C(NH₂)=NOH,
4,5-Dihydro-5-oxo-1,2,4-oxadiazol-3-yl,
4,5-Dihydro-5-thioxo-1,2,4-oxadiazol-3-yl,
2-Oxo-3H-1,2,3,5-oxathiadiazol-4-yl,
2,2-Dioxo-3H-1,2,3,5-oxathiadiazol-4-yl,
2,3-Dihydro-3-oxo-1,2,4-oxadiazol-5-yl,
2,5-Dihydro-2,5-dioxo-1H-imidazol-4-yl,
4,5-Dihydro-5-oxo-1H-1,2,4-triazol-3-yl,
4,5-Dihydro-5-thioxo-1H-1,2,4-triazol-3-yl,
2,3-Dihydro-2-oxo-1,3,4-thiadiazol-5-yl oder
4,5-Dihydro-5-oxo-1,2,4-thiadiazol-3-yl,
R³ H, R¹¹, unsubstituiertes oder ein- oder mehrfach durch COOH, COOA, CN, NO₂, NR⁹R¹⁰, NHCOR¹¹, NHSO₂R¹¹, Hal und/oder Ar substituiertes C₂-C₆-Alkenyl, C₂-C₆-Alkinyl, -CₙH₂ₙ-R¹², -CHR¹³-CₖH₂ₖ-R¹⁴ oder 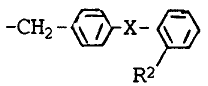
R⁴ H oder Hal,
R⁵ und R⁶ jeweils H, eine C₁-C₆-Alkylgruppe, worin auch eine CH₂-Gruppe durch O oder S ersetzt sein kann oder eine zusätzliche C-C-Doppelbindung enthalten sein kann und welche zusätzlich durch OH, OR⁷, Ar, Het², NR⁷R⁸, NR⁷-COOR⁸, NR⁷-COO-CₜH₂ₜ-Ar, NR⁷-COO-CₜH₂ₜ-Het² und/oder COOR⁷ substituiert sein kann, CF₃, C₃-C₈-Cycloalkyl, Ar oder Het²,
R⁷ und R⁸ jeweils A, C₂-C₆-Alkenyl, C₂-C₆-Alkinyl, C₃-C₈-Cycloalkyl-CₖH₂ₖ- oder C₁-C₆-Alkyl, worin eine CH₂-Gruppe durch O oder S ersetzt ist,
R⁹ und R¹⁰ jeweils H, A, C₂-C₆-Alkenyl oder C₂-C₆-Alkinyl, Ar, ArCₙH₂ₙ- oder Het²,
R⁹ auch -CH₂COOA, -SO₂-A oder -SO₂-Ar,
R⁹ und R¹⁰ zusammen auch eine Alkylenkette mit 2-5 C-Atomen, die ein- oder mehrfach durch Carbonylsauerstoff, Ar, Het², -CO-Ar, -COOA, -CO-N(A)₂, -CH₂OH, -SO₂-Ar und/oder -NH-CO-A substituiert und/oder durch O oder durch -NR¹⁹- unterbrochen sein kann,
R¹¹ C₁-C₅-Alkyl, worin auch ein oder mehrere H-Atom(e) durch F ersetzt sein können,
R¹² C₃-C₈-Cycloalkyl, CN, COOA, COOH, Ar, Het¹, Het², 1H-5-Tetrazolyl, -CO-NR⁹R¹⁰, -CO-R¹¹, -CO-Ar, -CO-Het², -CO-R¹⁷, -C(=NR¹⁵)-A, -C(=NR¹⁵)-Het², -S(O)ₘ-A, -S(O)ₘ-Ar, -S(O)ₘ-Het², -SO₂-NH-Het² oder -SO₂-OR¹⁸,
R¹³ COOH, COOA, CONR⁹R¹⁰, CN, NO₂, NHCOR¹⁴, NHSO₂R¹⁴ oder 1H-5-Tetrazolyl,
R¹⁴ Ar oder Cycloalkyl mit 3-8 C-Atomen,
R¹⁵ H, OH, CN, R¹⁶, OR¹⁶ oder OAr,
R¹⁶ A, C₂-C₆-Alkenyl oder C₂-C₆-Alkinyl,
R¹⁷ -NH-CHR¹⁸-COOH, -NH-CHR¹⁸-COOA, -CH₂S(O)ₘ-Ar, -CH₂ -COOA,-CₙH₂ₙ-NO₂, -CₙH₂ₙ-NR⁹R¹⁰ oder -CₙH₂ₙ-NHCOOA,
R¹⁸ H oder A,
R¹⁹ H, A, Ar, COOA, Het² oder SO₂-Ar,
X fehlt, -NH-CO- oder -CO-NH-,
Y O oder S,
A C₁-C₆-Alkyl,
Ar eine unsubstituierte oder eine durch R¹¹, OH, OR¹¹, COOH, COOA, CN, NO₂, NH₂, NHA, N(A)₂, NHCOR¹¹, NHCOOA, NHSO₂R¹¹, Hal und/oder 1H-5-Tetrazolyl mono- oder disubstituierte Phenylgruppe,
Het¹ einen fünf- oder sechsgliedrigen gesättigten heterocyclischen Rest mit 1 bis 3 N-, O- und/oder S-Atomen, der einfach durch Carbonylsauerstoff oder =NR¹⁵ und/oder dessen Ring-N-Atom(e) jeweils durch A oder Ar substituiert sein kann (können),
Het² einen fünf- oder sechsgliedrigen heteroaromatischen Rest mit 1 bis 3 N-, O- und/oder S-Atomen, der auch mit einem Benzol- oder Pyridinring kondensiert sein kann,
Hal F, Cl, Br oder I,
k 0, 1, 2, 3 oder 4,
m 0, 1 oder 2,
n 1, 2, 3, 4, 5 oder 6 und
t 1, 2, 3 oder 4 bedeuten,
sowie ihre Salze.

2. 2-Butyl-3-(2'-(4,5-dihydro-5-oxo-1,2,4-oxadiazol-3-yl)-biphenylyl-4-methyl)-4-oxo-5-benzyl-4,5-dihydro-3H-imidazo[4,5-c]pyridin.

3. Verfahren zur Herstellung von Imidazopyridinen der Formel I nach Anspruch 1 sowie ihrer Salze, dadurch gekennzeichnet, daß man
(a) eine Verbindung der Formel II 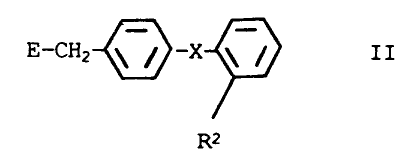 worin
E Cl, Br, I oder eine freie oder reaktionsfähig funktionell abgewandelte OH-Gruppe bedeutet und
R² und X die in Anspruch 1 angegebene Bedeutung haben, mit einer Verbindung der Formel III
H-R III
worin
R die in Anspruch 1 angegebene Bedeutung hat,
umsetzt
oder
(b) eine Verbindung der Formel IV 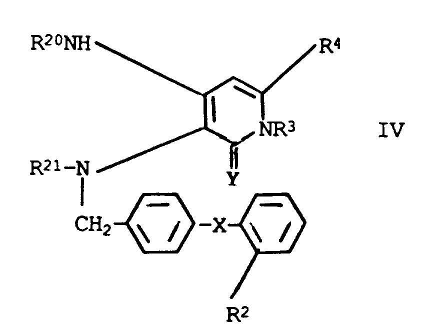 worin
R²⁰ R¹-CO oder H und
R²¹ H (falls R²⁰ R¹-CO ist) oder R¹-CO (falls R²⁰ H ist)
bedeuten
und
R¹, R², R³, R⁴, X und Y die in Anspruch 1 angegebenen Bedeutungen haben,
mit einem cyclisierenden Mittel behandelt,
oder
(c) zur Herstellung einer Verbindung der Formel I, worin X -NH-CO- oder -CO-NH- bedeutet, eine Verbindung der Formel V 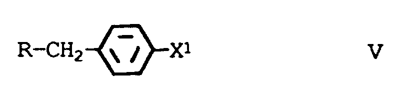 worin
X¹ NH₂ oder COOH bedeutet und
R die in Anspruch 1 angegebene Bedeutung hat
oder ein reaktionsfähiges Derivat dieser Verbindung mit einer Verbindung der Formel VI 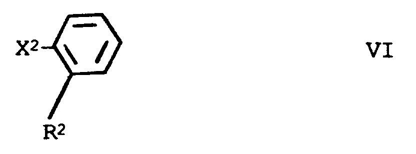 worin
X² COOH (falls X¹ NH₂ ist) oder NH₂ (falls X¹ COOH ist) bedeutet und
R² die in Anspruch 1 angegebene Bedeutung hat,
oder mit einem reaktionsfähigen Derivat dieser Verbindung umsetzt oder
(d) zur Herstellung einer Verbindung der Formel I, worin R² -C(NH₂)=NOH bedeutet, eine Verbindung, die der Formel I entspricht, aber an Stelle des Restes R² eine CN-Gruppe trägt, mit Hydroxylamin umsetzt oder
(e) zur Herstellung einer Verbindung der Formel I, worin R² -SO₂NH-COOR⁵, -SO₂NH-COR⁵, -SO₂NH-SO₂R⁵ oder -SO₂NH-CONR⁵R⁶ bedeutet, eine Verbindung, die der Formel I entspricht, aber an Stelle des Restes R² eine -SO₂NH₂-Gruppe trägt, mit einer Verbindung der Formel E-COOR⁵, E-COR⁵, E-SO₂R⁵, E-CONR⁵R⁶ oder O=C=NR⁵ umsetzt oder
(f) eine Verbindung der Formel I aus einem ihrer funktionellen Derivate durch Behandeln mit einem solvolysierenden oder hydrogenolysierenden Mittel in Freiheit setzt,
und/oder daß man in einer Verbindung der Formel I einen oder mehrere Rest(e) R, R² und/oder R³, in einen oder mehrere andere Reste R, R² und/oder R³ umwandelt und/oder eine Base oder Säure der Formel I in eines ihrer Salze umwandelt.

4. Verfahren zur Herstellung pharmazeutischer Zubereitungen, dadurch gekennzeichnet, daß man eine Verbindung der Formel I nach Anspruch 1 und/oder eines ihrer physiologisch unbedenklichen Säureadditionssalze zusammen mit mindestens einem festen, flüssigen oder halbflüssigen Träger- und Hilfsstoff in eine geeignete Dosierungsform bringt.

5. Pharmazeutische Zubereitung, gekennzeichnet durch einen Gehalt an mindestens einer Verbindung der Formel I nach Anspruch 1 und/oder einem ihrer physiologisch unbedenklichen Säureadditionssalze.

6. Verbindung der Formel I nach Anspruch 1 und ihre physiologisch unbedenklichen Säureadditionssalze zur Bekämpfung von Krankheiten.

7. Verwendung von Verbindungen der Formel I nach Anspruch 1 und/oder ihrer physiologisch unbedenklichen Säureadditionssalze zur Herstellung eines Arzneimittels.

8. Verwendung von Verbindungen der Formel I nach Anspruch 1 und/oder ihrer physiologisch unbedenklichen Säureadditionssalze bei der Bekämpfung von Krankheiten.
